# FASCICULE DE BREVET EUROPEEN

(11) **EP 1 009 767 B1**
(45) Date de publication et mention de la délivrance du brevet: **08.12.2004**
(21) Numéro de dépôt: 98942796.8
(22) Date de dépôt: 28.08.1998
(51) Int. Cl.: C07K 14/47, C12N 15/12, C12N 15/63, C07K 16/18, A61K 38/17, G01N 33/00

(54) **DEFENSINE HUMAINE DEF-X, GENE ET CDNA, COMPOSITION LES CONTENANT ET APPLICATIONS AU DIAGNOSTIC ET A LA THERAPIE**
MENSCHLICHE DEFENSIN DEF-X, GEN UND CDNA, DEREN ENTHALTENDE ZUSAMMENSETZUNGEN UND DEREN DIAGNOSTISCHE UND THERAPEUTISCHE ANWENDUNGEN
HUMAN DEFENSIN DEF-X, GENE AND DNAc, COMPOSITION CONTAINING SAME AND DIAGNOSTIC AND THERAPEUTIC APPLICATIONS

(30) Priorité: 29.08.1997 FR 9710823
(43) Date de publication de la demande: 21.06.2000
(73) Titulaire: GENSET, 91000 Evry (FR)
(72) Inventeur: BOUGUELERET, Lydie, F-92170 Vanves (FR); CHUMAKOV, Ilya, F-77000 Vaux-le-Penil (FR)
(74) Mandataire: Warcoin, Jacques
(86) Numéro de dépôt international: PCT/FR1998/001864
(87) Numéro de publication internationale: WO 1999/011663

(56) Documents cités:
- WO-A-89/11291
- WO-A-94/21672
- WO-A-95/32287
- US-A- 5 242 902
- US-A- 5 641 497
- WILDE C G ET AL: "PURIFICATION AND CHARACTERIZATION OF HUMAN NEUTROPHIL PEPTIDE 4, A NOVEL MEMBER OF THE DEFENSIN FAMILY" JOURNAL OF BIOLOGICAL CHEMISTRY, vol. 264, no. 19, 5 juillet 1989, pages 11200-11203, XP000027256

## Description

La présente invention concerne une nouvelle défensine polypeptidique humaine Def-X, homologue de l'HNP-4, son ADN génomique et ADNc.

L'invention concerne également des vecteurs de clonage et d'expression, des cellules transformées par lesdits vecteurs. L'invention a aussi pour objet l'utilisation desdits polypeptides comme agent antibiotique, cytotoxique, de réparation et de régulation endocrine ou comme pesticide ainsi que des compositions cosmétiques ou pharmaceutiques pour le traitement des infections microbiennes, notamment bactériennes, fongiques, et virales, ou parasitaires, de cancers, de l'inflammation et de déficit immunitaire. Enfin, l'invention comprend des méthodes et des kits de diagnostic pour la détermination d'une infection microbienne ou parasitaire et d'une inflammation, ou pour le dépistage de prédisposition à des déficiences immunitaires ou des maladies cancéreuses.

Les substances antimicrobiennes sont des éléments primordiaux de la défense des organismes multicellulaires. Parmi ces substances, on trouve aussi bien des composés inorganiques simples (péroxyde d'hydrogène, acide hypochloreux, oxyde nitrique) que des peptides et protéines complexes. Ils sont présents sur les premières lignes de défense, à la surface des muqueuses de différents organes, notamment dans les cellules épithéliales de l'intestin et des poumons, selon les espèces, ainsi que dans les organelles microbicides des cellules phagocytaires d'origine hématopoïétique, où ils furent tout d'abord mis en évidence. Leur synthèse *de novo* ou leur libération à partir de sites de stockage - organelles de type lysosomes, granules cytoplasmiques, capables de les stocker sous une forme inactive ou latente - peuvent être induites rapidement, ce qui les rend particulièrement importants dans les phases précoces de résistance aux infections (Martin et al., 1995).

Les protéines antimicrobiennes d'une taille inférieure à cent acides aminés sont arbitrairement appelées peptides antimicrobiens. Plusieurs families de peptides antimicrobiens ont été identifiées, qui diffèrent quant à la présence en leur sein de ponts disulfures, quant à leur composition en acides aminés, à leur conformation structurelle et à leur spectre d'activité. Les peptides antimicrobiens comportant six cystéines conservées forment la famille des défensines. Cette famille est composée de peptides antimicrobiens présents dans de nombreuses espèces, abondants, d'environ 3-4 kDa (Ganz et Lehrer, 1994). Ces peptides sont formés de 30 à 40 acides aminés, dont six cystéines invariantes qui forment trois liens disulfides intrameléculaires. Ils ont une conformation complexe, sont amphipathiques, riches en feuillets bêta anti-parallèles, mais dépourvus d'hélices alpha (Lehrer et Ganz, 1992). L'action antimierobienne des défcnsincs résulterait de leur insertion dans les membranes des cellules cibles, permettant la formation de canaux voltage-dépendants. White et al. (1995) décrivent les mécanismes possibles d'insertion membranaire et de formation de pores multimériques par les défensines, qui permettent la perméabilisation des membranes des cellules cibles, par exemple des cellules microbiennes ou tumorales. La structure cristallographique de la défensine humaine de neutrophile HNP-3 (voir ci-dessous) a été déterminée, et un mécanisme particulier de dimérisation des défensines humaines de neutrophile est en outre suggéré. La connaissance élargie de cette famille de peptides et la comparaison de leurs séquences et spectres d'activité permettront de mieux comprendre ces mécanismes et leurs spécificités, ainsi que les résidus acides aminés plus particulièrement impliqués dans ces phénomènes.

Les défensines se répartissent en trois familles de peptides, structurellement différents : les défensines "classiques", les bêta-défensines et les défensines des insectes. Ces familles présentent des différences concernant la position et l'espacement des résidus cystéines conservés, ainsi que ceux d'autres acides aminés conservés (proline, glycine) (Ganz et Lehrer, 1995).

Les défensines humaines, de type classique, proviennent essentiellement de deux sources. Elles ont d'abord été identifiées par purification peptidique à partir d'extraits de neutrophiles. Quatre défensines ont ainsi été isolées: "human neutrophil peptides" HNP-1, HNP-2, HNP-3, et HNP-4. Les trois premières sont des produits différents du même gène (Ganz et Lehrer, 1995). Ces trois peptides représentent 99 % du contenu des neutrophiles en défensines, alors que HNP-4 y est aussi présent, mais à des concentrations 100 fois plus faibles. Plus récemment, deux défensines entériques humaines, HD-5 et HD-6, ont été caractérisées dans l'intestin grêle et plus précisément dans les cellules de Paneth (Bevins et al., 1996). Alors que 16 gènes de défensines entériques ont été mis en évidence chez la souris, seuls ces deux homologues ont été identifiés chez l'homme (Mallow et al., 1996).

Les défensines ont une action antimicrobienne sur un large spectre de microorganismes *in vitro* (Martin et al., 1995). Ce spectre d'action, particulièrement large, comprend des bactéries, Gram-positives et Gram-négatives, plusieurs champignons, des mycobactéries, des parasites dont les spirochètes et plusieurs virus à enveloppe dont les virus HSV et HIV. Elles sont également cytotoxiques pour plusieurs catégories de cellules normales et malignes, dont les cellules résistantes au TNF-alpha et au facteur cytolytique NK (Kagan et al., 1994). La grande quantité de cibles des défensines et leur abondance dans les cellules sanguines spécialisées dans la défense immunitaire, ainsi que l'augmentation dramatique de leur concentration au cours d'infections sévères, suggèrent que ces molécules joueraient un rôle important dans l'immunité naturelle aux infections et aux cancers. Notamment, l'augmentation de la transcription des gènes des défensines et la libération de granules cytoplasmiques contenant des défensines pré-synthétisées en réponse à des stimuli, contribuent à la réponse antimicrobienne locale, les défensines pouvant participer à la réaction d'inflammation, aux processus de réparation et à la régulation endocrine pendant l'infection. Les défensines hématopoïétiques pourraient contribuer au phénomène de lyse des cellules cancéreuses, phénomène médié par les neutrophiles au cours de la réponse immunitaire anticorps-dépendante. Le rôle physiologique précis des défensines entériques n'est pas clairement établi. Elles pourraient endiguer la prolifération de la flore intraluminale ou empêcher la translocation de bactéries à travers la muqueuse intestinale (Mallow et al., 1996). L'abondance de l'ARNm de défensine dans les cellules de Paneth renforce l'hypothèse que ces cellules épithéliales joueraient un rôle clé dans la défense immunitaire de l'intestin. Il a par ailleurs été montré que leur schéma d'expression coïncide avec l'apparition des cellules de Paneth au cours de l'embryogenèse. Mallow et al. (1996) ont suggéré que de faibles taux d'expression de défensines entériques chez le foetus serait le témoin d'une immaturité de la défense locale, ce qui prédisposerait les enfants nés prématurément à des infections dues aux microorganismes intestinaux.

Une concentration des défensines correspondant à 10 % du taux normal est constatée chez des patients atteints de "specific granule deficiency", une maladie rare du développement des granulocytes. Les sujets atteints souffrent d'infections fréquentes, provoquées par des bactéries communes (Ganz et Lehrer, 1995).

Les défensines modifiées biochimiquement sont de potentiels agents prophylactiques et thérapeutiques contre les infections (Ganz et Lehrer, 1995). La recherche concernant ces peptides antimicrobiens ou d'autres molécules participant de l'immunité naturelle, acquiert une importance particulière depuis que se développent des phénomènes de résistance des microorganismes aux antibiotiques traditionnels (Bevins et al., 1996).

La structure primaire de défensines, notamment des défensines humaines, a fait l'objet d'études récentes (White et al., 1995 ; Mallow et al., 1996). Les défensines classiques comprennent 29 à 35 acides aminés, mais dérivent de précurseurs - préproprotéines - comprenant 90 à 100 acides aminés. La maturation protéolytique des défensines humaines de neutrophiles en peptides matures est couplée avec leur adressage vers les granulocytes ; la fonction du propeptide inclurait l'inactivation de la forme précurseur de la défensine et un support à l'acquisition de la conformation active du peptide mature (Martin et al., 1995). Les homologies peptidiques sont maximales au niveau des signaux peptides, et minimales au niveau des peptides matures, qui comportent néanmoins six résidus cystéines totalement conservés. Si la conservation de ces résidus semble nécessaire à l'acquisition de structures secondaires impliquées dans l'activité des défensines, les différences de séquences existant au sein de la très large famille de ces peptides antimicrobiens, notamment à leur extrémité N-terminale, mais aussi dans d'autres régions non conservées, semblent être des déterminants importants de leur spectre d'activité, et de leur efficacité antimicrobienne ou cytotoxique. L'identification de nouveaux membres de cette famille de peptides, et notamment de défensines humaines, est donc nécessaire à la compréhension de leur mécanisme d'action et de leur spécificité, ainsi qu'à leur utilisation comme agents anti-infectieux et/ou cytotoxiques, ou au dessin de peptides variants présentant des spectres spécifiques et/ou d'efficacité diminuée ou augmentée.

Sparkes et al. (1989), ont localisé le gène codant pour HNP-1 sur le chromosome 8, dans la région 8p23. Bevins et al. (1995), et Mallow et al. (1996), ont localisé les deux gènes codant pour HD-5 et HD-6 sur le chromosome 8, plus précisément dans la région 8p21-pter, région incluant la région précédemment identifiée comme portant les défensines hématopoïétiques. Les gènes codant pour les défensines entériques humaines HD-5 et HD-6 contiennent deux exons, alors que ceux codant pour les défensines hématopoïétiques en contiennent trois, les deux derniers exons codant pour le prépropeptide, aussi bien chez l'homme, que chez le cobaye et le lapin (Mallow et al., 1996). La comparaison des séquences génomiques des gènes *HD-5* et *HD-6* a révélé une très forte similarité des séquences flanquantes non codantes en 5', suggérant que celles-ci contiennent l'information nécessaire à la tissu-spécificité de l'expression de ces gènes; ces mêmes régions portent en outre de nombreux sites de fixation pour des facteurs de transcription, dont deux sites AP2 et six sites IL6, suggérant des voies de régulation de l'expression de ces gènes au cours des processus inflammatoires. De façon plus générale, le très important degré de similarité des séquences et de l'organisation génomique des défensines HNP-1, 2, 3, 4 et HD-5 et 6, a conduit Bevins et al. (1995) à un modèle d'évolution tentant de relater l'organisation chromosomique de la famille, et les fractions homologues de chaque paire de gènes.

Il est enfin intéressant de noter que la région chromosomique 8p23 est impliquée dans de nombreuses pathologies, notamment cancéreuses : on citera par exemple le carcinome hépatocellulaire (Becker et al., 1996), le cancer du poumon non à petites cellules (Sundareshan et Augustus, 1996), le cancer de la prostate (Ichikawa et al., 1996), et le carcinome colorectal (Yaremko et al., 1994). Bien que ceci n'ait jamais été documenté, il est possible qu'une déficience en l'une ou l'autre des défensines humaines ait un rôle dans la prédisposition à de telles pathologies, ou dans leur développement.

La présente invention concerne une nouvelle défensine humaine, Def-X, homologue de la défensine HNP-4.

La présente invention a donc pour objet un polypeptide isolé choisi parmi les polypeptides suivants :
a) polypeptide dont la séquence d'acides aminés est la séquence SEQ ID N° 6 ;
b) polypeptide) présentant au moins 80% d'identité avec le polypeptide dont la séquence d'acides aminés est la séquence SEQ ID N° 6;
c) polypeptide dont la séquence d'acides aminés est la séquence d'acides aminés d'un fragment biologiquement actif d'au moins 10 acides aminés consécutifs d'un polypeptide tel que défini en a) ou b) ;
d) polypeptide comprenant au moins un polypeptide tel que défini en a), b) ou c).

Dans la présente description, on entendra désigner également par « polypeptide » une protéine ou un peptide.

Selon un mode préféré, le polypeptide selon l'invention est caractérisé en ce qu'il est constitué du polypeptide de séquence SEQ ID N°3.

On peut également citer ici la polypeptides constitués de l'un au moins des fragments suivants:
a) peptide signal dont la séquence d'acides aminés est la séquence SEQ ID N° 4, correspondant à la séquence comprise entre la position 1 et la position 19, extrémités comprises, de la séquence d'acides aminés SEQ ID N° 3 ;
b) région pro dont la séquence d'acides aminés est la séquence SEQ ID N° 5, correspondant à la séquence comprise entre la position 20 et la position 63, extrémités incluses, de la séquence d'acides aminés SEQ ID N° 3 ;
c) peptide mature dont la séquence d'acides aminés est la séquence SEQ ID N° 6, correspondant à la séquence comprise entre la position 64 et la position 94, extrémités incluses, de la séquence d'acides aminés SEQ ID N° 3 ; ou
d) fragment homologue, variant ou modifié d'un peptide selon a), b) ou c).

De façon encore préférée, les polypeptides selon la présente invention correspondent à la structure primaire de la défensine mature définie précédemment, c'est-à-dire la structure correspondant a la séquences d'acides aminés SEQ ID N° 6 suivante :
Ile Cys His Cys Arg Val Leu Tyr Cys Ile Phe Gly Glu His Leu Gly Gly Thr Cys
Phe Ile Leu Gly Glu Arg Tyr Pro Ile Cys Cys Tyr,
ses homologues présentant au moins 80% d'identité ainsi que leurs fragments biologiquement actifs et les polypeptides les contenant.

Il est bien entendu que les polypeptides de l'invention sont sous forme non naturelle, c'est-à-dire qu'ils ne sont pas pris dans leur environnement naturel mais qu'ils ont pu être obtenus par purification à partir de sources naturelles ou bien obtenus par recombinaison génétique ou par synthèse chimique comme cela sera décrit ci-après.

Par « polypeptide homologue », on entend un polypeptide dont la séquence d'acides aminés présente au minimum 80 %, et préférentiellement 90 %, d'acides aminés en commun.

Par « polypeptide variant », on entend désigner un polypeptide muté ou correspondant à un polymorphisme pouvant exister, notamment chez l'être humain et pouvant présenter une troncature, une substitution, une délétion et/ou une addition d'au moins un acide aminé comparé au polypeptide selon l'invention.

Par « polypeptide modifié », on entend désigner un polypeptide obtenu par recombinaison génétique ou par synthèse chimique comme cela sera décrit ci-après, présentant une modification par rapport à la séquence normale. Ces modifications pourront notamment porter sur les domaines pré-, pro- ou mature du polypeptide selon l'invention, sur les acides aminés à l'origine d'une spécificité de spectre ou d'efficacité de l'activité, ou à l'origine de la conformation structurale, de la charge, ou de l'hydraphobicité, et de la capacité de multimérisation et d'insertion membranaire du polypeptide selon l'invention. On pourra ainsi créer des polypeptides d'activité équivalente, augmentée ou diminuée, et de spécificité équivalente, plus étroite, ou plus large. Les modifications pourront aussi porter sur les séquences impliquées dans la maturation, le transport et l'adressage du polypeptide.

Par « fragment biologiquement actif » d'un polypeptide selon l'invention, on entend désigner un fragment polypeptidique ayant conservé au moins une activité du polypeptide dont il est issu, en particulier :
- capable d'être reconnu par un anticorps spécifique d'un polypeptide selon l'invention ; et/ou
- capable d'agir comme antibiotique ; et/ou
- capable d'agir comme agent cytotoxique ; et/ou
- capable d'agir comme agent antitumoral ; et/ou
- capable de moduler la réparation de tissu, la régulation endocrine ou le processus d'inflammation, notamment durant une infection.

Selon l'invention, les fragments biologiquement actifs de polypeptides selon l'invention auront au minimum 10 acides aminés, de préférence 15 acides aminés.

Comme cela a été indiqué précédemment, parmi les fragments biologiquement actifs, un fragment préféré est le peptide mature de séquence d'acides aminés SEQ ID N° 6.

Parmi les homologues du peptide mature, il faut citer les polypeptides dans lesquels jusqu'à 5 acides aminés ont été modifiés, tronqués à l'extrémité N- ou C-terminale, ou bien délétés, ou bien ajoutés, ce qui représente environ 80 % de la séquence.

Les fragments biologiquement actifs de ce peptide mature comportent de préférence de 10 à 15 acides aminés, dont l'intérêt pourra être de pouvoir être obtenus facilement par synthèse chimique.

Comme cela est indiqué, les modifications du polypeptide mature auront pour objectif notamment de :
- modulcr l'activité de la défensine,
- modifier sa spécificité, tant au niveau des microorganismes sur lesquels elle est active que sur sa localisation tissulaire,
- modifier sa biodisponibilité.

Les composés précédents peuvent être obtenus en utilisant la chimie combinatoire, dans laquelle il est possible de faire varier systématiquement des parties de polypeptide avant de les tester sur des modèles, cultures cellulaires ou des microorganismes par exemple, pour sélectionner les composés les plus actifs ou présentant les propriétés recherchées.

La synthèse chimique présente également l'avantage de pouvoir utiliser :
- des acides aminés non naturels, ou
- des liaisons non peptidiques.

Ainsi, afin d'améliorer la durée de vie des peptides, il pourra être intéressant d'utiliser des acides aminés non naturels, par exemple sous forme D, ou bien des analogues d'acides aminés, notamment des formes soufrées par exemple.

Enfin, la structure de la défensine mature ou de ses homologues, variants ou modifiés, de même que les fragments correspondant, pourront être intégrés dans des structures chimiques de type polypeptidique ou autres. Ainsi, il pourra être intéressant de prévoir aux extrémités N- et C-terminales des composés non reconnus par les protéases.

L'invention comprend également les acides nucléiques isolés codant pour un polypeptide selon l'invention.

Selon un mode préféré, les acides nucléiques selon l'invention seront choisis parmi les acides nucléiques suivants :
a) acide nucléique de séquence SEQ ID N° 1 (génomique) ;
b) acide nucléique de séquence SEQ ID N° 2 (cDNA) ;
c) acide nucléique présentant au moins 80% d'identité, par rapport aux acides nucléiques selon a) ou b) ;
d) fragment de la séquence SEQ ID N°1 comprenant au moins un exon de séquence nt 1836 à 1874, nt 3394 à 3577 ou nt 4164 à 4379 de la séquence SEQ ID N°1.

Il est entendu que la présente invention ne concerne pas les séquences génomiques dans leur environnement chromosomique naturel ; il s'agit de séquences qui ont été isolées, c'est-à-dire qu'elles ont été prélevées directement ou indirectement, leur environnement ayant été au moins partiellement modifié.

Il peut ainsi s'agir d'ADN génomique, d'ADNc, ou d'ARN, comportant ou non des nucléotides non naturels ; il peut s'agir d'acides nucléiques naturels isolés, ou d'acides nucléiques de synthèse.

Par acide nucléique équivalent, on entendra un acide nucléique codant pour les polypeptides selon l'invention, compte tenu de la dégénérescence du code génétique, et les ADNc et ARN correspondants.

Par acide nucléique homologue, on entendra un acide nucléique dont la séquence présente une homologie d'au moins 80 %, de préférence 90 %, avec les séquences nucléiques selon l'invention.

Par acide nucléique muté, on entendra tout acide nucléique codant pour un polypeptide variant selon l'invention, et tout acide nucléique comportant, par rapport aux séquences SEQ ID N° 1 et SEQ ID N° 2, au moins une mutation dans les séquences promotrices et/ou régulatrices, lesquelles pourront avoir un effet sur l'expression du polypeptide notamment sur son taux d'expression et la tissu-spécificité de celle-ci. Les séquences présentant un polymorphisme présent chez l'être humain sont donc incluses dans l'invention. Parmi ces polymorphismes, certains pourront conduire à des déficiences immunitaires, de réponse aux infections, à des prédispositions et/ou au développement de cancers.

Par acide nucléique modifié, on entendra tout acide nucléique codant pour un polypeptide modifié selon l'invention, ou tout acide nucléique obtenu par mutagenèse selon des techniques bien connues de l'homme de l'art, et comportant des modifications par rapport aux séquences normales, notamment des mutations dans les séquences régulatrices et/ou promotrices, notamment conduisant à une modification du taux et/ou de la tissu-spécificité de l'expression du polypeptide.

La présente invention concerne l'ensemble des amorces et sondes, qui pourront être marquées selon des méthodes bien connues de l'homme du métier, permettant de mettre en évidence, notamment par des techniques basées sur l'hybridation ou sur l'amplification, par exemple par PCR, les séquences nucléiques selon l'invention, y compris de discriminer les séquences normales des séquences mutées.

Parmi les fragments d'acides nucléiques intéressants, il faut citer en particulier les oligonucléotides anti-sens, c'est-à-dire dont la structure assure, par hybridation avec la séquence cible, une inhibition de l'expression du produit correspondant. Il faut encore citer les oligonucléotides sens qui, par interaction avec des protéines impliquées dans la régulation de l'expression du produit correspondant, induiront soit une inhibition, soit une activation de cette expression.

Il pourra s'agir de séquences qui agissent aussi bien au niveau des séquences exoniqucs ou introniques décrites que sur les séquences flanquantes, notamment les promoteurs et/ou régions 5' UTR.

La présente invention concerne également des vecteurs de clonage ou d'expression comportant une séquence nucléotidique telle que décrite précédemment.

Ces vecteurs de clonage ou d'expression pourront comporter des éléments assurant l'expression de la séquence dans une cellule hôte, notamment des séquences promotrices et des séquences de régulation efficaces dans ladite cellule.

Le vecteur en cause pouvant être à réplication autonome ou bien destiné à assurer l'intégration de la séquence au sein des chromosomes de la cellule hôte.

Dans le cas de systèmes à réplication autonome, en fonction de la celluie hôte, procaryote ou eucaryote, on utilisera de préférence des systèmes de type plasmidique ou des systèmes viraux, les virus vecteurs pouvant être notamment des adénovirus (Perricaudet et al., 1992), des rétrovirus, des poxvirus ou des virus herpétiques (Epstein et al., 1992). L'homme de métier connaît les technologies utilisables pour chacun de ces virus.

Ainsi, il est connu d'utiliser comme vecteur viral des virus défectifs dont la culture est effectuée dans des cellules de complémentation, ceci évitant les risques éventuels de prolifération d'un vecteur viral infectieux.

Lorsque l'on souhaitera l'intégration de la séquence dans les chromosomes de la cellule hôte, il sera nécessaire de prévoir de part et d'autre de la séquence nucléotidique à intégrer une ou plusieurs séquences provenant de la cellule hôte afin d'assurer la recombinaison. Il s'agit là également de procédés qui sont largement décrits dans la technique antérieure. On pourra, par exemple, utiliser des systèmes de type plasmidique ou viral ; de tels virus seront, par exemple, les rétrovirus (Temin, 1986) ou les AAV, Adenovirus Associated Virus (Carter, 1993).

L'invention concerne également les cellules procaryotes ou eucaryotes transformées par un vecteur tel que décrit précédemment et ceci afin d'assurer l'expression d'une défensine Def-X naturelle, normale ou variante, ou modifiée, ou bien, par exemple, d'un de ses fragments.

Comme cela a été indiqué précédemment, la présente invention concerne également les polypeptides obtenus par culture des cellules ainsi transformées et récupération de la protéine exprimée, ladite récupération pouvant être effectuée de façon intracellulaire ou bien de façon extracellulaire dans le milieu de culture lorsque le vecteur a été conçu pour assurer la sécrétion de la protéine par le biais, par exemple, d'une séquence "signal", le polypeptide étant sous forme d'un pré-polypeptide ou prépro-polypeptide. Les constructions permettant la sécrétion des polypeptides sont connues, aussi bien pour des systèmes procaryotes que des systèmes eucaryotes. Dans le cadre de la présente invention, certains des polypeptides Def-X pourront comporter leur propre système de sécrétion ou d'insertion membranaire.

Il est bien entendu que les polypeptides recombinants selon l'invention peuvent être obtenus sous forme glycosylée ou non glycosylée et présenter ou non la structure tertiaire naturelle.

Parmi les cellules utilisables pour la production de ces polypeptides, il faut citer bien entendu les cellules bactériennes (Olins et Lee, 1993), mais également les cellules de levure (Buckholz, 1993), de même que les cellules animales, en particulier les cultures de cellules de mammifère (Edwards et Aruffo, 1993) mais également les cellules d'insectes dans lesquelles on peut utiliser des procédés mettant en oeuvre des baculovirus par exemple (Luckow, 1993).

Les cellules ainsi obtenues peuvent permettre de préparer des polypeptides naturels, variants ou modifiés, Def-X, mais également des fragments de ces polypeptides, notamment des polypeptides pouvant correspondre aux fragments biologiquement actifs.

La présente invention concerne, en outre, les mêmes polypeptides selon l'invention mais obtenus par synthèse chimique et pouvant comporter des acides aminés non naturels ou modifiés.

Les polypeptides selon la présente invention, en particulier la défensine mature, de même que les homologues, dérivés ou polypeptides matures modifiés, peuvent être obtenus par synthèse chimique et ce en utilisant l'une quelconque des nombreuses synthèses peptidiques connues, par exemple les techniques mettant en oeuvre des phases solides ou des techniques utilisant des phases solides partielles, par condensation de fragments ou par une synthèse en solution classique.

Lorsque les composés selon la présente invention sont synthétisés par la méthode en phase solide, l'acide aminé C-terminal est fixé sur un support solide inerte et comporte des groupes protecteurs de son groupement amino en alpha (et si cela est nécessaire, des protections sur ses groupes fonctionnels latéraux).

A la fin de cette étape, le groupe protecteur du groupement amino terminal est éliminé et on fixe le second acide aminé comportant lui aussi les protections nécessaires.

Les groupes protecteurs N-terminaux sont éliminés après que chaque acide aminé a été fixé, par contre on maintient, bien entendu, la protection sur les chaînes latérales.

Lorsque la chaîne polypeptidique est complète, on clive le peptide de son support et on élimine les groupes de protection latéraux.

La technique de synthèse en phase solide est décrite notamment dans Stewart et al. (1984) et Bodanszky (1984).

Il ne sera pas ici évoqué les détails de la synthèse, il convient simplement de rappeler que les groupes protecteurs préférés pour les groupements alpha-amino sont des groupes protecteurs de type uréthane (BOC ou FMOC). Quant aux réactifs de couplage, ils sont très nombreux, parmi eux il faut bien entendu citer plus particulièrement la N,N'-diisopropyl-carbodiimine (DIC) mise en oeuvre en général dans le DMF ou le DCM.

Lorsque l'on souhaitera utiliser des amino-acides non naturels, il pourra être nécessaire de prévoir d'autres types de réactif et en particulier d'autres types de système de protection.

La présente invention concerne également les anticorps polyclonaux ou monoclonaux obtenus par réaction immunologiquc d'un organisme humain ou animal avec un agent immunogène constitué par un polypeptide selon l'invention, notamment un polypeptide obtenu par culture d'une des cellules précédemment décrites, ou par synthèse chimique comme indiqué précédemment.

L'invention s'étend donc aux anticorps monoclonaux et polyclonaux ou un de leurs fragments, anticorps chimériques, capables de reconnaître spécifiquement un polypeptide selon l'invention.

L'invention comprend aussi les anticorps selon l'invention, caractérisés en ce qu'ils sont marqués.

Les anticorps marqués pourront être, par exemple, immunoconjugués à des enzymes telles que la péroxydase ou la phosphatase alcaline, ou marqués à l'aide de composés fluorescents, de la biotine ou encore radiomarqués. Les techniques de marquage sont bien connues de l'homme du métier et ne seront pas développées dans la présente description.

L'invention s'étend également à un polypeptide selon l'invention pour utilisation comme agent antimicrobien, notamment antibactérien, antifongique, antiviral et/ou antiparasitaire, comme agent cytotoxique, à visée notamment anticancéreuse, et/ou comme agent de modulation des processus d'inflammation, de réparation tissulaire et de régulation endocrine, notamment corticostatique.

Selon un autre aspect, l'invention concerne une composition pharmaceutique comprenant un polypeptide selon l'invention, pouvant être associée à un véhicule pharmaceutiquement acceptable.

Une telle composition pourra être administrée par voie systémique, locale ou topique.

Son mode d'administration, sa posologie, ses formes galéniques optimales pourront être déterminés selon les critères généralement pris en compte dans l'établissement d'un traitement adapté à un patient, notamment son âge, son poids corporel, la tolérance de traitement, ses effets secondaires constatés, etc..

L'invention comprend également une composition pharmaceutique comprenant un vecteur selon l'invention capable d'exprimer *in vivo* un polypeptide selon l'invention, pouvant être associé à un véhicule pharmaceutiquement acceptable.

Il est également possible de prévoir l'expression de polypeptides ou leurs fragments *in vivo,* notamment par le biais de la thérapie génique et en utilisant les vecteurs qui ont été décrits précédemment.

Dans le cadre de la thérapie génique, il est possible également de prévoir l'utilisation des séquences des gènes ou des ADNc précédemment décrits, "nus", cette technique a notamment été développée par la société Vical, qui a montré qu'il était, dans ces conditions, possible d'exprimer le polypeptide dans certains tissus sans avoir recours au support d'un vecteur viral notamment.

Toujours dans le cadre de la thérapie génique, il est également possible de prévoir l'utilisation de cellules transformées ex-vivo, lesquelles pourront être ensuite réimplantées, soit telles quelles, soit au sein de systèmes de type organoïde, tel que cela est également connu dans l'état de la technique (Danos et al. 1993). On peut également envisager l'utilisation d'agents facilitant le ciblage d'un type cellulaire déterminé, la pénétration dans les cellules ou le transport vers le noyau.

Lesdites compositions pharmaceutiques sont, selon l'invention, destinées à la prévention et/ou au traitement des infections microbiennes, notamment les infections microbiennes d'origines bactériennes, de bactéries Gram-positives ou Gram-négatives, mycobactériennes, fongiques et virales, ou parasitaires, notamment de spirochètes.

Selon un mode préféré, l'invention concerne avantageusement les compositions pharmaceutiques selon l'invention caractérisées en ce que les infections virales sont des infections liées à des virus à enveloppe, notamment les virus HSV et HIV.

L'invention a également pour objet des compositions pharmaceutiques selon l'invention, destinées à la prévention et/ou au traitement des cancers, notamment les mélanomes, le cancer du foie, de la prostate, du poumon non à petites cellules ou le carcinome colorectal.

L'invention comprend, en outre, des compositions pharmaceutiques selon l'invention, destinées à augmenter les défenses immunitaires, à augmenter les défenses immunitaires en cas d'immunodéficience acquise ou à prévenir l'immunodéficience, notamment pour le traitement du psoriasis, ou à moduler les processus inflammatoires dans les cas notamment de maladies à inflammation chronique.

Les polypeptides selon la présente invention sont plus particulièrement utilisables sous forme topique externe, par exemple sur la peau et les muqueuses. Ces formes topiques externes peuvent être aussi bien à usage pharmaceutique, dermatologique qu'à usage cosmétique.

En particulier, ces compositions peuvent être utilisées comme agent antiseptique pharmaceutique ou bien comme antiseptique dans certains cosmétiques, soit pour assurer un nettoyage de la peau ou des phanères et/ou à titre de conservateur des compositions.

Les compositions topiques selon la présente invention peuvent être utilisées notamment dans certaines affections cutanées, oculaires, vaginales ou buccales. Elles peuvent également être utilisées comme agent cosmétique additionnel, notamment dans certains shampooings traitants.

L'invention concerne également la mise en évidence de l'absence ou d'une quantité anormale de protéine ou d'acide nucléique correspondant à la défensine X comme marqueur d'une infection ou de pathologies qui seront décrites ci-après.

L'invention concerne également la mise en évidence d'une forme anormale de la protéine ou la présence d'un acide nucléique anormal correspondant à une défensine mutée qui peut éventuellement être totalement inactive. Dans ce cas, la présence de cette forme anormale peut être un marqueur de prédisposition à certaines affections, notamment l'immunodéficience et/ou des cancers.

C'est pourquoi, la présente invention concerne une méthode de diagnostic d'une immunodéficience et/ou d'une prédisposition à certains types de cancers, caractérisée en ce qu'on met en évidence dans un prélèvement de patient la présence d'une défensine anormale et/ou d'une séquence codant pour une défensine anormale.

Les méthodes de diagnostic selon la présente invention permettent, notamment, la mise en évidence d'une immunodéficience, et/ou d'une prédisposition à un ou des cancers, notamment ceux cités précédemment, en particulier dans des familles à risque. Ce type de diagnostic sera en général effectué par mise en évidence des formes mutées de la protéine ou des séquences d'acide nucléique.

Mais l'invention concerne également des méthodes de diagnostic de l'inflammation, d'immunodéficience, de prédisposition à des affections de type cancer et/ou d'infections dues à des microorganismes ou liées à un déficit immunitaire ou phénomène inflammatoire, caractérisées en ce qu'elles comprennent le dosage d'un polypeptide ou d'un acide nucléique selon l'invention dans un échantillon biologique et la comparaison du résultat dudit dosage obtenu avec la quantité de polypeptide ou d'acide nucléique présente normalement dans un échantillon biologique équivalent.

Dans ce cas, le dosage peptidique permettra, en général, une détection d'une infection microbienne ou parasitaire et/ou d'une inflammation. Les dosages peptidiques peuvent être réalisés par tout procédé connu, ELISA ou RIA par exemple. La mise en évidence d'une forme anormale de la défensine-X peut être réalisée, par exemple, à l'aide d'un anticorps monoclonal spécifique de cette forme, en particulier les anticorps objet de l'invention.

Selon un mode de réalisation préféré, l'invention comprend avantageusement les méthodes caractérisées en ce qu'elles mettent en oeuvre une sonde et/ou une amorce oligonucléotidique selon l'invention.

On préfèrera en général les méthodes dans lesquelles tout ou partie de la séquence correspondant au polypeptide Def-X est amplifiée préalablement par dosage d'acide nucléique selon l'invention, ces méthodes d'amplification pouvant être réalisées par des méthodes dites PCR ou PCR-like. Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; lorsque l'échantillon d'origine est un ARN il convient préalablement d'effectuer une transcription réverse. Il existe actuellement de très nombreux procédés permettant cette amplification, par exemple les méthodes dites NASBA "Nucleic Acid Sequence Based Amplification" (Compton 1991), TAS "Transcription based Amplification System" (Guatelli et al. 1990), LCR "Ligase Chain Reaction" (Landegren et al. 1988), "Endo Run Amplification" (ERA), "Cycling Probe Reaction" (CPR), et SDA "Strand Displacement Amplification" (Walker et al. 1992), bien connues de l'homme du métier.

L'invention concerne en outre des kits ou nécessaires de diagnostic pour la détermination d'une infection microbienne ou parasitaire, d'une inflammation, d'une immunodéficience et/ou d'une prédisposition à des affections de type cancer, caractérisés en ce qu'ils comprennent un anticorps selon l'invention.

Les kits ou nécessaires de diagnostic pour la détermination d'une infection microbienne ou parasitaire, d'une inflammation, d'une immunodéficience et/ou de prédisposition à des affections de type cancer, caractérisés en ce qu'ils comprennent une sonde et/ou une amorce selon l'invention font également partie de l'invention.

L'invention a, enfin, pour objet l'utilisation de polypeptide selon l'invention comme pesticide, notamment pour la culture de végétaux d'intérêt industriel comme, par exemple, les plantes vivrières telles que le maïs, le blé, le soja, le riz ou le colza, les plantes fourragères, les arbres fruitiers, la vigne ou les plantes ornementales.

D'autres caractéristiques et avantages de la présente invention apparaîtront à la lecture des exemples ci-après, illustrés par les figures dont les légendes sont décrites ci-dessous.

### Légendes des figures

### Figure 1

### Séquence génomique de hDef-X.

Est présentée la totalité de la séquence d'ADN génomique de hDef-X qui présente une homologie significative avec le gène codant pour hDef-4 (HNP-4).

La séquence présente les sites suivants, dont la présence est déduite par homologie avec la séquence hDef-4 :
- CAAT box 1711-1714
- TATA box 1758-1767
- mRNA start 1836
- exon 1 1836-1874
- site d'épissage 1 GTCAGT
- insertion Alu 2155-2335
- insertion fragment de L1 2710-2780
- site d'épissage 2 CAG
- exon 2 3394-3577
- début de phase codante 3406
- site d'épissage 3 GTGAGA
- site d'épissage 4 CAG
- exon 3 4164-4379
- fin de phase codante 4276
- site de polyadénylation 4374-4379.

### Figure 2

Alignement des séquences génomiques des défensines humaines Def-X et Def-4 (HNP-4).

Alignement de la totalité de la séquence d'ADN génomique de la nouvelle défensine Def-X présentant une homologie avec l'ADN génomique de hDef-4 (GenBank accession number U18745).

Les annotations présentent les positions sur la séquence de hDef-4 des signaux CAAT box, TATA box, sites d'épissage, débuts et fins d'introns/d'exons, début de transcription, site de polyadénylation.

### Figure 3

### Alignement des séquences d'ADNc de hDef-4 (HNP-4) et hDef-X.

Les séquences présentent une homologie globale de 61,4 %. L'alignement révèle une insertion d'environ 75 bases en aval du codon STOP, présentes sur la séquence de hDef-4, mais non sur celle de hDef-X ; l'homologie forte reprend sur toute la région comprise entre l'extrémité de cette insertion et celle de l'ADNc. En dehors de cette région d'insertion, le degré d'homologie entre séquences nucléiques est donc remarquable.

### Figure 4

### Séquence peptidique de la protéine hDef-X.

La position des sites de clivage du signal peptide et de la région pro ont été déduites de l'alignement des séquences peptidiques de hDef-4 et hDef-X.

### Figure 5

Alignement des séquences peptidiques des défensines humaines connues hDef-1, hDef-4, hDef-5, et hDef-6 avec hDef-X.
* L'étoile indique un acide aminé conservé sur les cinq séquences.
• Le point indique un acide aminé dont la classe est conservée sur les cinq séquences (acide aminé soit identique, soit faisant l'objet d'une substitution conservative).
^ six flèches indiquent les positions des six cystéines conservées au travers de la classe des défensines classiques et responsables de la structure tridimensionnelle nécessaire à l'activité de ces peptides.

### EXEMPLES

### Exemple 1 : Identification du gène codant pour hDef-X

### Isolement du BAC B0725B12

Afin d'analyser la région 8p23 du génome humain, notamment dans la région connue comme portant des gènes codant pour des défensines humaines, on a isolé un BAC ("Bacterial Artificial Chromosome") correspondant à ladite région. Une banque de BACs couvrant le génome humain complet a été préparée à partir de l'ADN d'une lignée lymphoblastique humaine dérivée de l'individu n° 8445 des familles du CEPH. Cette lignée a été utilisée comme source d'ADN de haut poids moléculaire. L'ADN a été partiellement digéré par l'enzyme de restriction BamH1, puis clone au site BamH1 du plasmide pBeloBacII. Les clones ainsi obtenus ont été "poolés" et criblés selon une procédure d'analyse tridimensionnelle précédemment décrite pour le criblage des banques de YACs ("Yeast Artificial Chromosome") (Chumakov et al., 1992 et 1995). Les pools tridimensionnels obtenus ont été criblés par PCR à l'aide des amorces encadrant le marqueur SHGC-10793, pour Neutrophil defensin 4 precursor (GeneBank : numéro d'accession U18745) ; un clone du BAC B0725 B12 a été ainsi isolé.

Après digestion par l'enzyme de restriction NotI, la taille de l'insert porté par ce BAC a été déterminée sur un gel d'agarose 0,8 % après migration par électrophorèse en champ alterné (CHEF) (4 heures à 9Volts/cm, avec un angle de 100°, à 11°C en tampon 0,5 x TAE). On a ainsi mis en évidence que le BAC B0725B12 porte un insert de 220 kb, avec un site interne pour l'enzyme NotI.

### Localisation chromosomique du BAC B0725B12 par hybridation in situ fluorescente (FISH)

La localisation chromosomique du BAC dans la région candidate 8p23.1-23.2 a été confirmée par hybridation in situ fluorescente (FISH) sur chromosomes métaphasiques, selon la méthode décrite par Cherif et al., (1990).

### Séquençage de l'insert du BAC B0725B12

Afin de séquencer l'insert du BAC B0725B12, on a préparé une banque de sous-clones à partir de l'ADN soniqué de ce BAC.

Les cellules issues d'un litre de culture "overnight" ont été traitées par lyse alcaline selon les techniques classiques. Après centrifugation du produit obtenu dans un gradient de chlorure de césium, 12 µg d'ADN du BAC B0725B12 ont été purifiés. 3 µg d'ADN ont été soniqués afin d'obtenir des fragments dont les tailles se distribuent uniformément de 1,2 kb à 1,5 kb. Les fragments obtenus ont été traités dans un volume de 50 µl avec 2 unités de Vent polymérase pendant 20 minutes à 70°C, en présence des 4 déoxytriphosphates (100 µM). Les fragments aux extrémités franches résultant de cette étape ont été séparés par électrophorèse en gel 1 % d'agarose à bas point de fusion (60 Volts pendant 3 heures). Les fragments groupés selon leurs tailles ont été excisés et les bandes obtenues traitées par l'agarose. Après extraction au chloroforme et dialyse sur colonnes Microcon 100, l'ADN en solution a été ajusté à une concentration de 100 ng/µl. Une ligation a été effectuée "overnight" en mettant en présence 100 ng de l'ADN fragmenté du BAC B0725B12 et 20 ng d'ADN du vecteur BluescriptSK linéarisé par digestion enzymatique, et traité par la phosphatase alcaline. Cette réaction a été réalisée dans un volume final de 10 µl en présence de 40 unités/µl de T4 ADN ligase (New England Biolabs). Les produits de ligation ont ensuite servi à transformer par électroporation, soit une souche XL-Blue (pour les plasmides multicopies), soit une souche D10HB (pour les sous-clones issus du BAC). Les clones IacZ⁻ résistant à l'antibiotique ont été repiqués individuellement en microplaques pour stockage et séquençage.

On a ainsi obtenu 960 sous-clones correspondant à l'insertion de fragments de 1,2 kb à 1,5 kb au site BamHI (rendu franc) du plasmide BluescriptSK.

Les inserts de ces sous-clones ont été amplifiés par PCR sur cultures bactériennes conduites "overnight", en utilisant les amorces des vecteurs flanquant les insertions. La séquence des extrémités de ces inserts (en moyenne 500 bases de chaque côté) a été déterminée par séqucnçage automatique fluorescent sur séquenceur ABI 377, équipé du logiciel ABI Prism DNA Sequencing Analysis (version 2.1.2).

Les fragments de séquence provenant des sous-BACs ont été assemblés par le logiciel Gap4 de R. Staden (Bonfield et al., 1995). Ce logiciel permet la reconstruction d'une séquence complète à partir de fragments de séquences. La séquence déduite de l'alignement des différents fragments est la séquence consensus.

On a enfin utilisé des techniques de séquençage dirigé (marche systématique de l'amorce) pour parfaire les séquences et relier les contigs.

### Analyse des séquences pour l'identification de gènes

Les exons potentiels de l'insert du BAC B0725B12 ont été repérés par recherche d'homologie sur les banques publiques de protéines, d'acides nucléiques et d'EST (Expressed Sequence Tags).

### Banques de données

On a utilisé des refontes locales des principales banques publiques. La banque de protéines utilisées est constituée par la fusion non redondante des banques Genpept (traduction automatique de GenBank, NCBI ; Benson et al., 1996) ; Swissprot (George et al., 1996) et PIR/NBRF (Bairoch et al., 1996). Les doublons ont été éliminés par le logiciel "nrdb" (domaine public, NCBI ; Benson et al., 1996). Les répétitions internes ont ensuite été masquées par le logiciel "xnu" (domaine public, NCBI ; Benson et al., 1996). La banque résultante, dénommée NRPU (Non-Redundant Protein-Unique) a servi de référence pour les recherches d'homologies protéiques. Les homologies trouvées avec cette banque ont permis de localiser des régions codant potentiellement pour un fragment de protéine au moins apparenté à une protéine connue (exons codants). La banque d'EST utilisée est composée des sous-sections "gbest" (1-9) de Genbank (NCBI ; Benson et al., 1996). Elle contient tous les fragments de transcrits publics.

Les homologies trouvées avec cette banque ont permis de localiser des régions potentiellement transcrites (présentes sur l'ARN messager).

La banque d'acides nucléiques (autres que les EST) utilisée contient toutes autres sous-sections de Genbank et de l'EMBL (Rodriguez-Tome et al., 1996) dont les doublons ont été éliminés comme précédemment.

### Logiciels

On a utilisé l'ensemble de logiciels BLAST (Altschul et al , 1990) de recherche d'homologies entre une séquence et des banques de données protéiques ou nucléiques. Les seuils de signification utilisés dépendent de la longueur et de la complexité de la région testée ainsi que de la taille de la banque de référence. Ils ont été ajustés et adaptés à chaque analyse.

### Exemple 2 : analyse des séquences nucléiques et peptidiques de hDef-X

### Structure du gène codant pour hDef-X

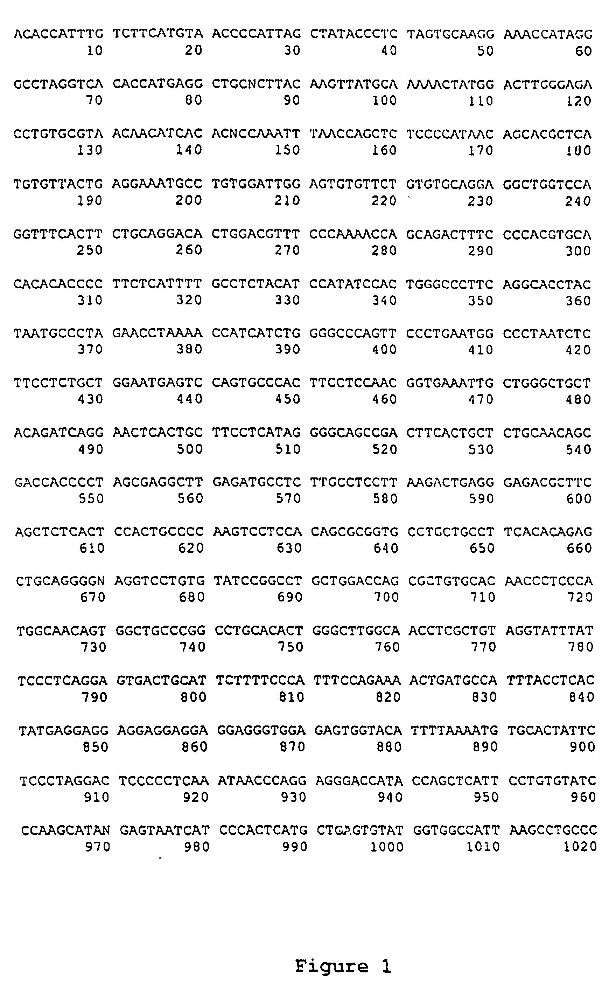

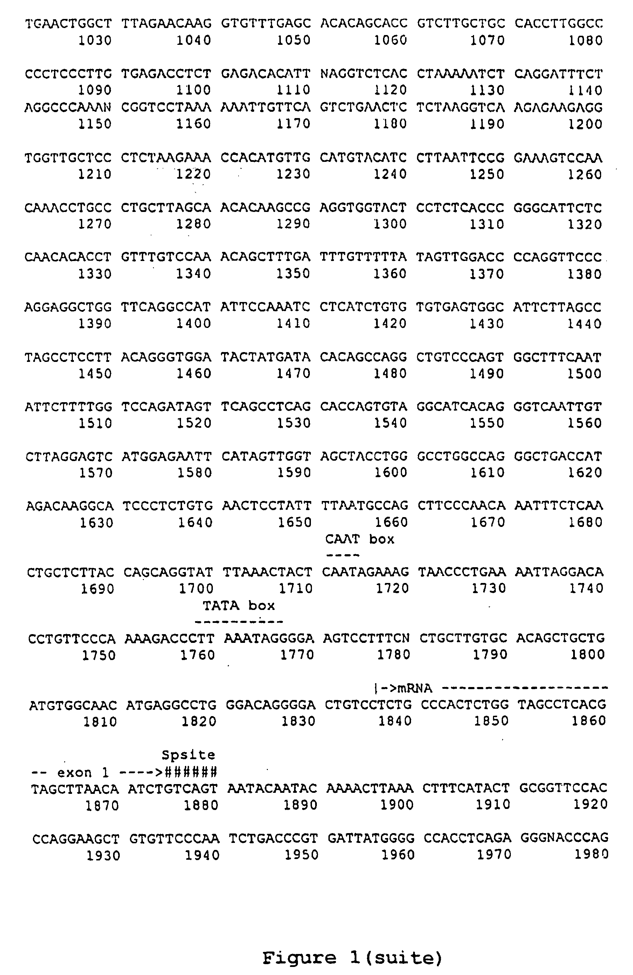

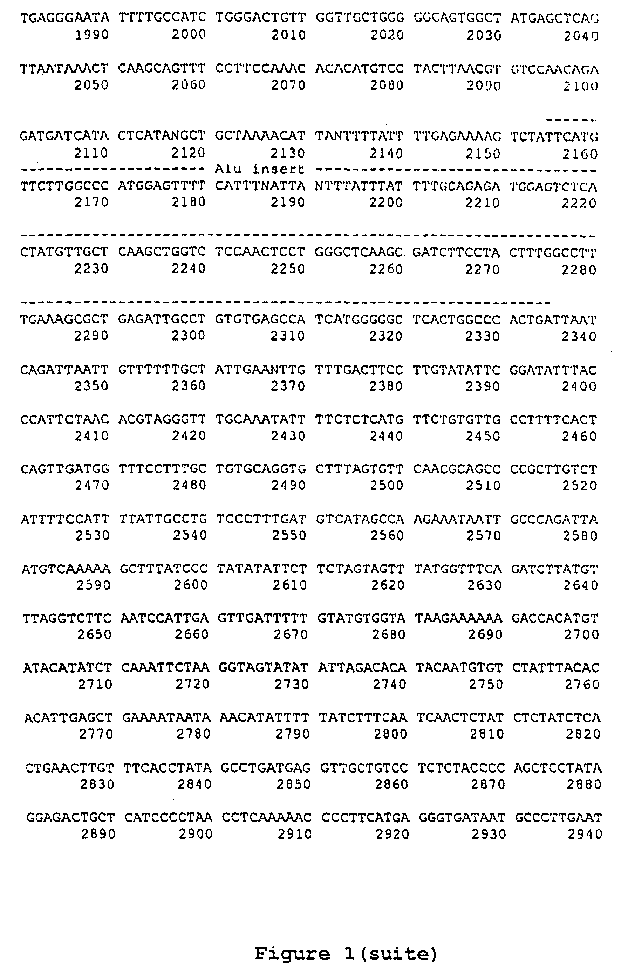

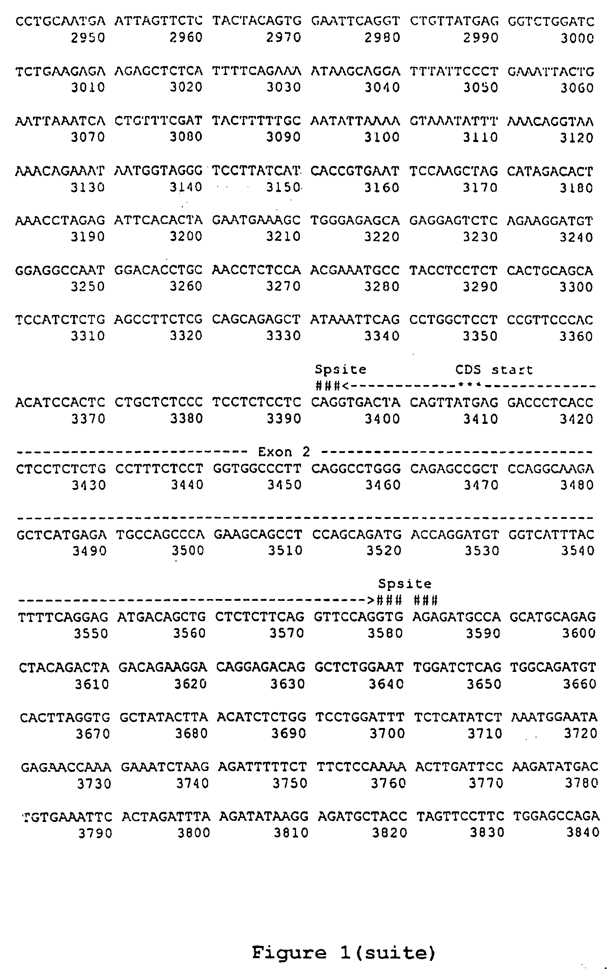

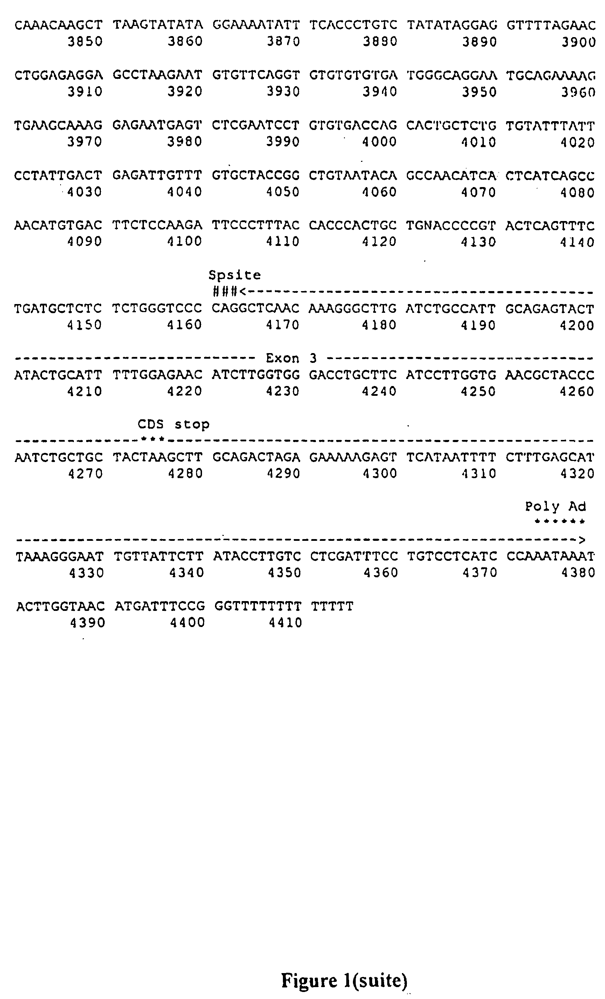

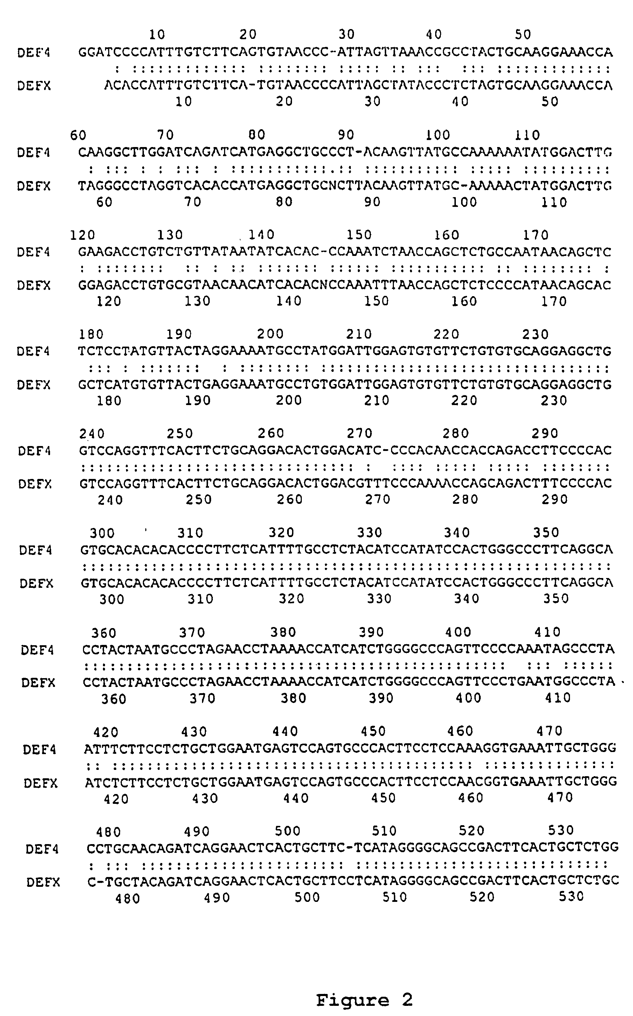

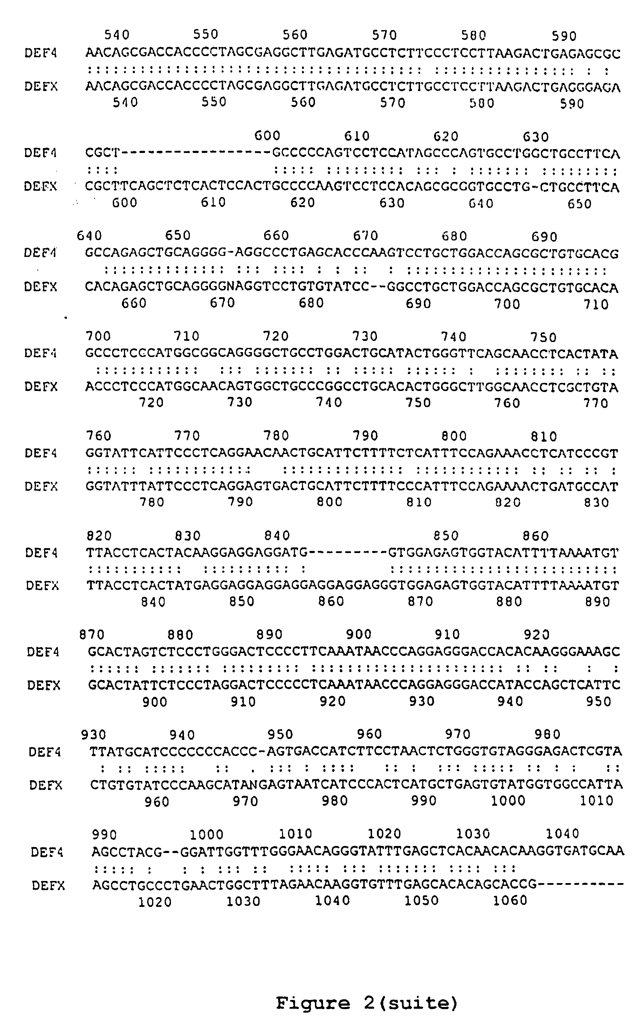

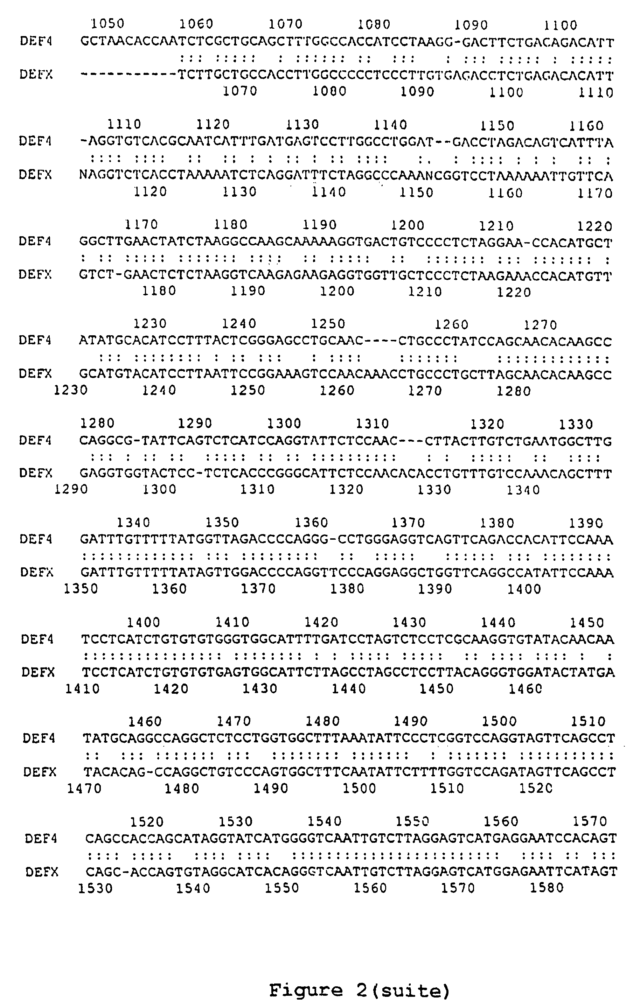

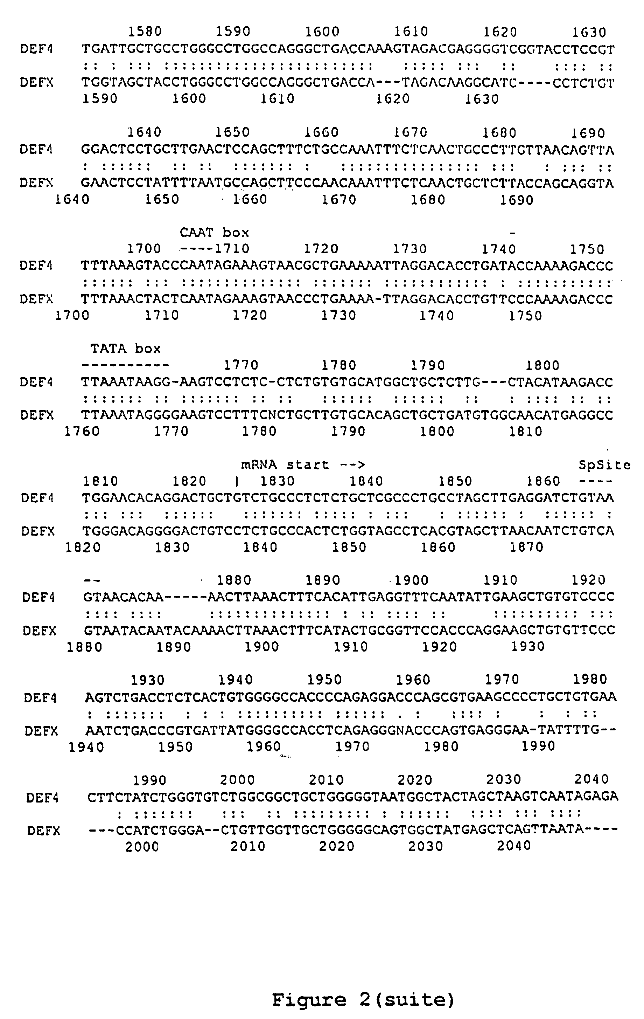

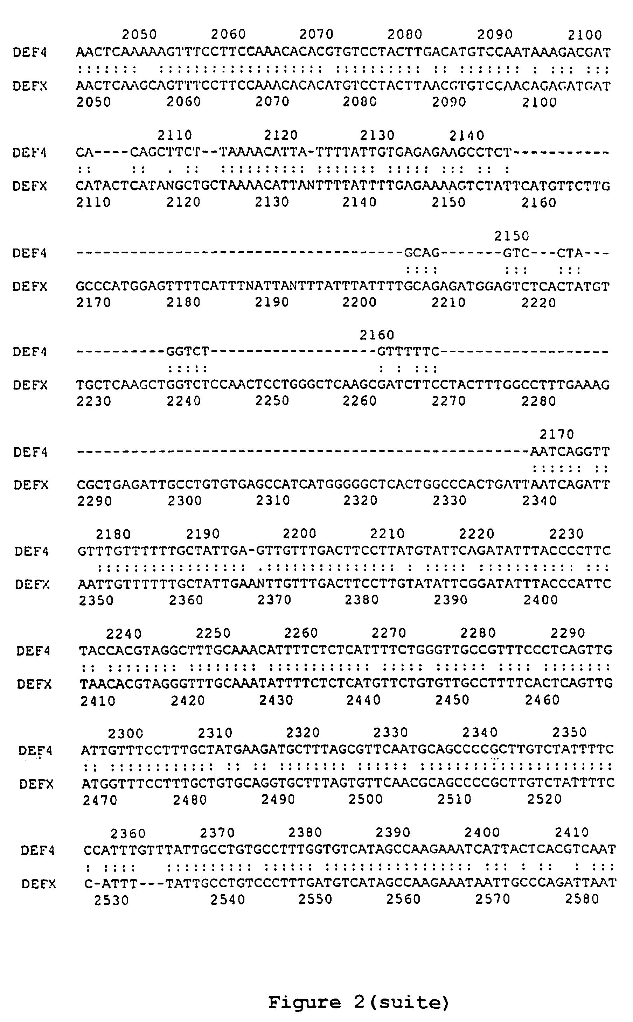

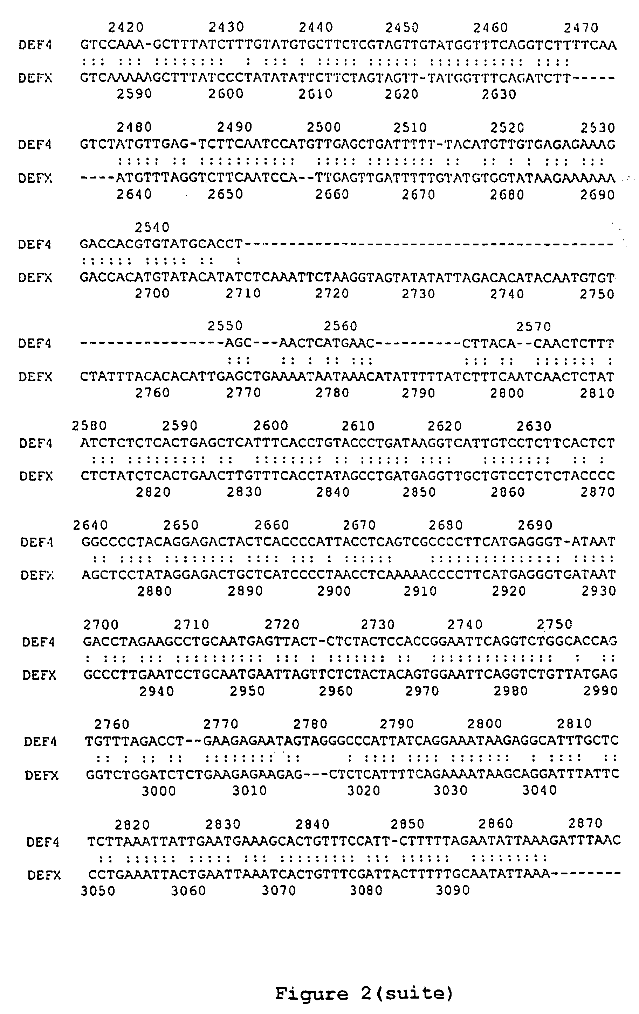

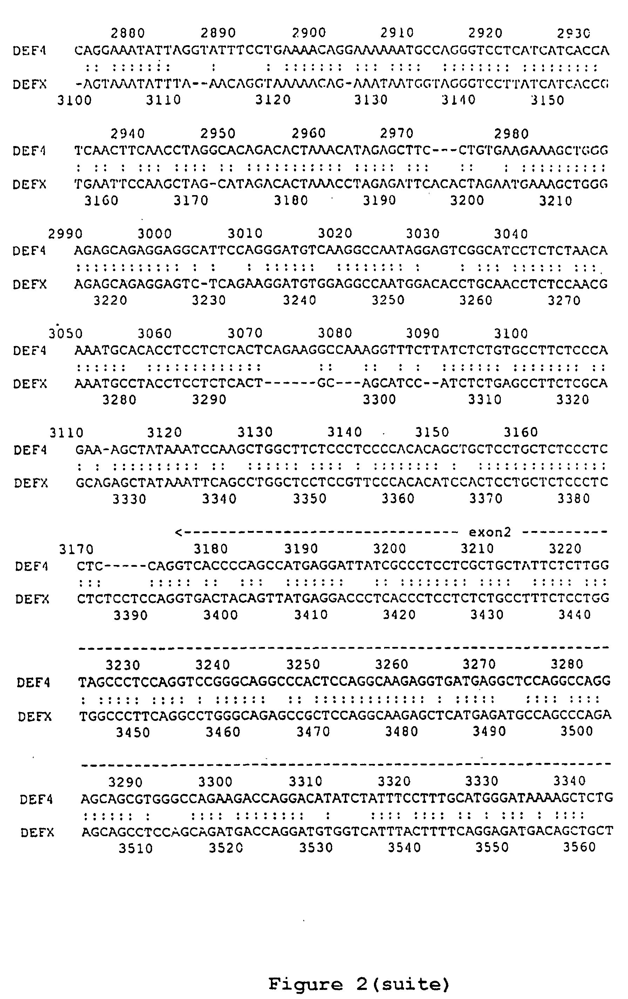

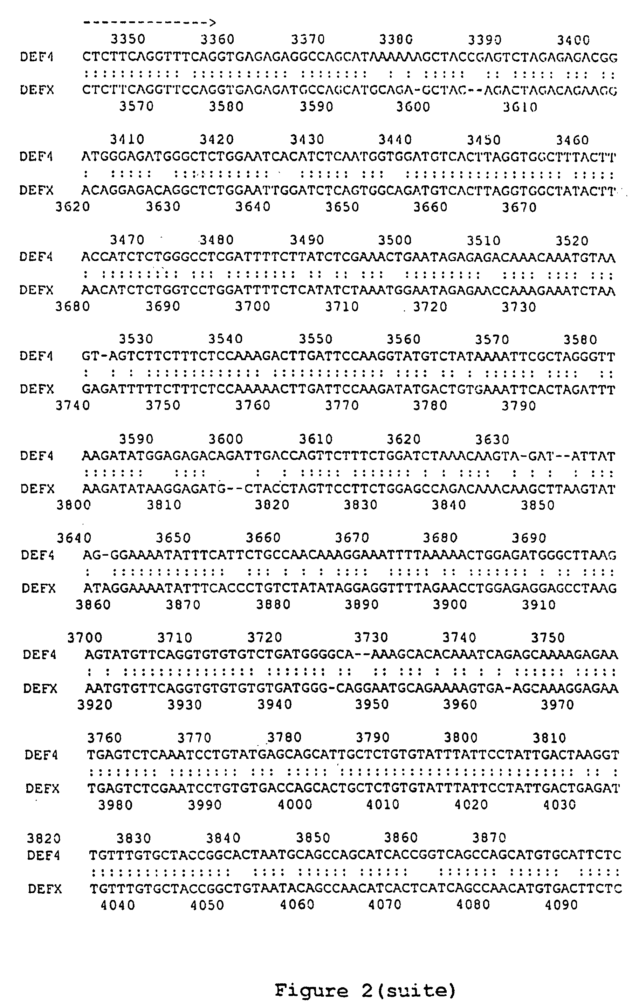

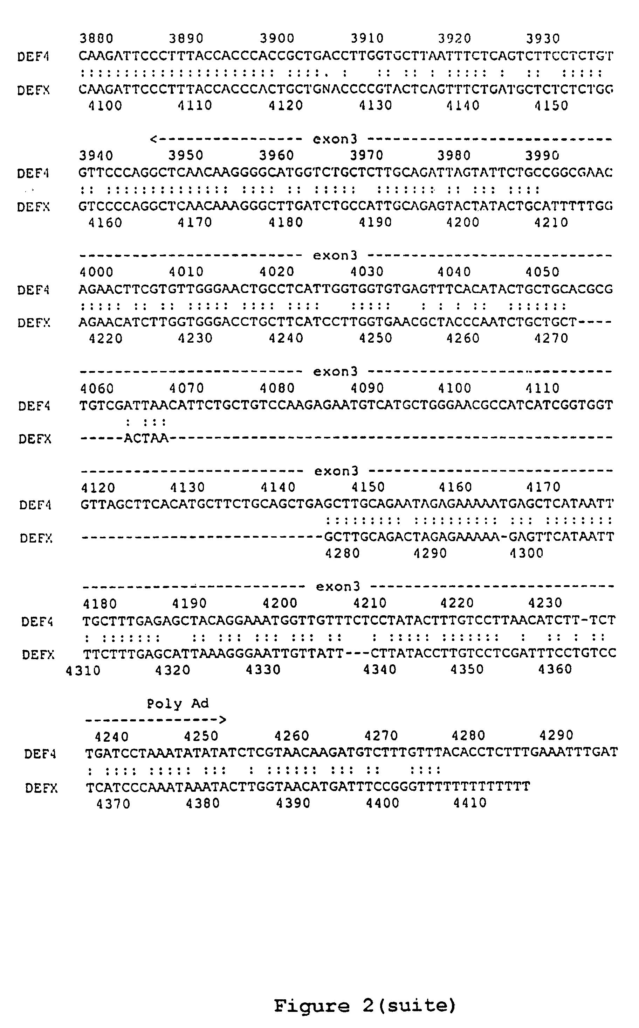

L'alignement du gène codant pour hDef-X avec ceux codant pour les défensines connues a permis de noter une homologie maximale entre hDef-X et hDef-4 (Figure 2). Le taux global d'homologie des deux séquences nucléiques est de 72 %. Les deux seules régions de l'ADN génomique de hDef-X ne présentant pas d'homologie avec celui de hDef-4 correspondent à deux zones d'insertion de séquence répétée dans la séquence de hDef-X, qui sont absentes sur la séquence de hDef-4 : un élément de type Alu (positions 2155 à 2335) et un fragment d'élément de Line 1 (positions 2710 à 2780).

On note une conservation importante de la région flanquant en 5' la région promotrice, d'où découle probablement une conservation importante des éléments de régulation de la stabilité du messager et de l'expression du gène.

La forte conservation de la séquence de l'exon 1, non traduit, permet de rattacher définitivement la défensine hDef-X à la classe des défensines classiques hématopoïétiques, soit hDef-1, 2, 3 et 4, par opposition aux défensines entériques hDef-5 et 6, dont la séquence génomique ne comporte que deux exons, tous deux codants.

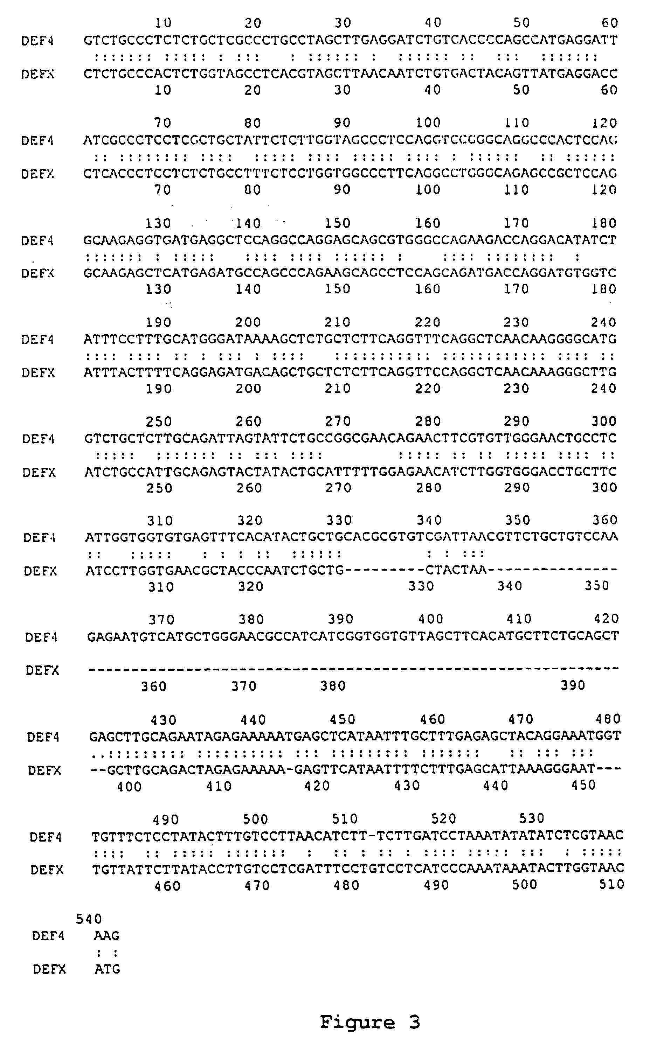

L'alignement des ADNc de hDef-4 et hDef-X, indiquant une homologie supérieure à 60 %, est présenté Figure 3.

### Analyse protéique

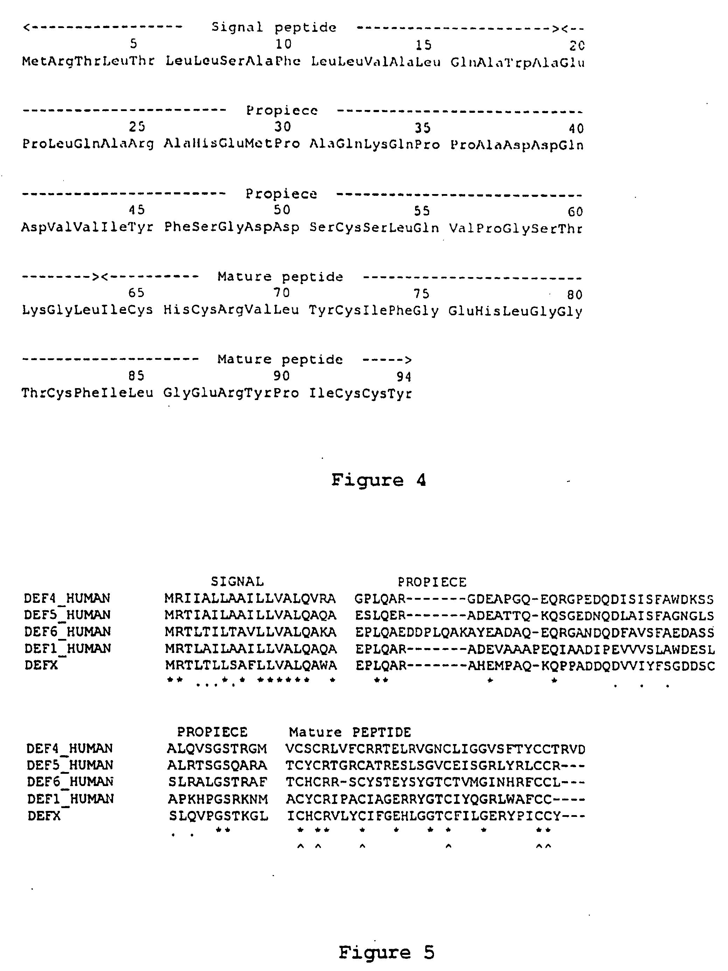

La séquence peptidique de la défensine selon l'invention est représentée Figure 4. Les trois domaines de la protéine sont positionnes comme suit :
- peptide signal : aa 1-19
- région pro : aa 20-63
- peptide mature : aa 64-94.

Les degrés d'homologies spécifiques entre hDef-4 et hDef-X ont été calculés, selon la région de la protéine concernée :
- peptide signal : 63,2 %
- région pro : 52,3 %
- peptide mature : 37,9 %.

L'homologie globale est de 49,5 %. Ces chiffres confirment la très forte homologie qui existe entre défensines, homologie maximale au niveau des peptides signaux et minimale au niveau des peptides matures.

On retrouve dans la séquence protéique primaire de Def-X les acides aminés conservés dans la classe des défensines classiques, notamment les six cystéines impliquées dans la structure tridimensionnelle de celles-ci (Figure 5).

Afin de prédire les structures secondaires présentes sur la défensine selon l'invention, on a utilisé les logiciels de prédiction de structure secondaire inclus dans le Protein Interpretation Package, Copyright MRC 1994, Medical Research Council, Hillsroad, Cambridge, United Kingdom.

Ces logiciels ont notamment permis de comparer les structures prédites de Def-X et HNP-4. Profils d'hydrophobicité, structures en alpha-hélices, feuillets β, amphiphilicité sont superposables dans les deux peptides, ce qui suggère des processus analogues d'insertion membranaire et de formation de canaux ioniques multimériques pour ces deux défensines.

### Exemple 3 : Recherche de mutations associées à des cas familiaux de cancers Extraction de l'ADN génomique

L'ADN génomique de patients immunodéficients ou atteints de cancer, est extrait du sang veineux périphérique après lyse cellulaire, digestion protéique, partition organique et finalement précipitation alcoolique, selon des techniques classiques bien connues de l'homme de l'art.

Il est notamment intéressant d'étudier la présence de mutations dans l'ADN génomique d'individus issus de familles à fort taux cancer, tous types de cancers confondus. Une déficience dans un gène de défensine de granulocyte, tel hDef-X peut en effet avoir un rôle dans la prédisposition aux cancers, comme mentionné précédemment.

### Amplification de l'ADN génomique

Des amorces oligonucléotidiques sont utilisées pour l'amplification génomique des séquences exoniques dérivées du BAC B0725B12 ; elles sont prédites par analyse informatique, et définies à l'aide du logiciel OSP (Hillier et al., 1991).

Toutes ces amorces contiennent, en amont des bases spécifiquement ciblées par l'amplification, une queue oligonucléotidique universelle commune, destinée à permettre le séquençage des fragments amplifiés (PU : 5'-TGTAAAACGACGGCCAGT-3' pour les amorces en amont, et RP : 5'-CAGGAAACAGCTATGACC-3' pour les amorces en aval).

Les amorces oligonucléotidiques sont synthétisées selon la méthode des phosphoramidites, sur un synthétiseur GENSET UFPS 24.1.

L'amplification de chaque séquence exonique prédite est réalisée par réaction d'amplification en chaîne par polymérase (PCR), dans les conditions suivantes :

| | |
|---|---|
| Volume final | 50 µl |
| ADN génomique | 100 ng |
| MgCl2 | 2 mM |
| dNTP (pour chacun) | 200 µM |
| Amorce (pour chacune) | 7.5 pmoles |
| AmpliTaq Gold DNA polymerase (Perkin) | 1 unité |
| Tampon de PCR (10X = 0.1 M Tris HCl pH 8.3, 0.5 M KCl) | 1 X. |

L'amplification est réalisée dans un thermocycleur Perkin Elmer 9600 ou MJ Research PTC200 avec couvercle chauffant. Après un chauffage à 94°C pendant 10 minutes, 35 cycles sont effectués. Chaque cycle comprend : 30 secondes à 94°C, 1 minute à 55°C et 30 secondes à 72°C. Un segment final d'élongation de 7 minutes à 72°C termine l'amplification.

La quantité de produits d'amplification obtenue est déterminée sur microplaque de 96 puits, par fluorométrie, utilisant l'agent intercalant Picogreen (Molecular Probes).

### Détection des polymorphismes/mutations

Les produits de l'amplification génomique par PCR sont séquencés sur séquenceur automatique ABI 377, en utilisant des amorces fluorescentes marquées par les fluorochromes ABI (Joe, Fam, Rox et Tamra) et l'ADN polymérase Thermosequanase (Amersham).

Les réactions sont réalisées en microplaques de 96 puits, sur thermocycleur Perkin Elmer 9600, dans des conditions classiques de cycles de température :
- 8 cycles : dénaturation : 5 sec. à 94°C ; hybridation : 10 sec. ; élongation : 30 sec. à 72°C, puis
- 13 cycles : dénaturation : 5 sec. à 94°C ; élongation : 30 sec. à 72°C.

6 unités de Thermosequanase, et 5-25 ng de produit d'amplification sont utilisés par réaction de séquence.

A l'issue des cycles d'amplification, les produits des réactions de séquence sont précipités dans l'éthanol, resuspendus dans du tampon de charge contenant de la formamide, dénaturés, et déposés sur gels d'acrylamide 4 % ; les électrophorèses (2 heures 30 à 3 000 Volts) sont conduites sur séquenceurs ABI 377 équipés des logiciels ABI de collection et d'analyse (ABI Prism DNA Sequencing Analysis Software, version 2.1.2.).

Les séquences obtenues chez des patients atteints des déficiences étudiées, notamment chez des patients issus de familles à forte prédisposition aux cancers, sont comparées aux séquences obtenues chez des sujets contrôles, apparentés et non apparentés. Une analyse statistique (calcul de lod score) permet de conclure quant à la signification de la présence d'un site d'hétérozygotie et à son association avec une prédisposition aux cancers.

### Exemple 4 : Recherche de mutations ponctuelles

Les mutations ponctuelles identifiées comme indiqué ci-dessus, peuvent ensuite être mises en évidence chez des sujets présentant une potentielle déficience dans le gène codant pour hDef-X, selon de nombreuses méthodes connues de l'homme de l'art. Parmi celles-ci, on peut citer la liste non exhaustive suivante :
- séquençage
- « single nucleotide primer extension » (Syvanen et al., 1990)
- RFLP
- recherche de « single strand conformation polymorphism »
- méthodes basées sur un clivage des régions misappariées (clivage enzymatique par la S1 nucléase, clivage chimique par différents composés tels que la pipéridine ou le tétroxide d'osmium)
- mise en évidence d'hétéroduplex en électrophorèse
- méthodes basées sur l'utilisation d'« allele specific oligonucleotide» (ASO, Stoneking et al., 1991)
- méthode OLA (« dual color oligonucleotide ligation assay, Samiotaki et al., 1994)
- méthode ARMS (« amplification refractory mutation system »), ou ASA (« allele specific amplification »), ou PASA (« PCR amplification of specific allele ») (Wu et al., 1989).

### REFERENCES

Altschul, Stephen F., Gish W., Miller W., Myers E. W., & Lipman D.J. Basic local alignment search tool. J. Mol. Biol. 215:403-10 (1990).

Bairoch A. & Apweiler R. The SWISS-PROT protcin sequence data bank and its new supplement TREMBL. Nucleic Acids. Res. 24: 21-25 (1996).

Becker S.A., Zou, Y.Z. & Slagle, B.L. Frequent loss of chromosome 8p in hepatitis B virus-positive hepatocellular carcinomas from China. Cancer Res. 56 (21) : 5092-7 (1996).

Benson D. A., Boguski M., Lipman D. J. & Ostell J. GenBank. Nucleic Acids Res. 24: 1-5 (1996).

Bodansky M., Principles of peptide synthesis, (1984).

Bevins, C.L., Joncs, D.E., Dutra, A., Schaffzin, J. & Muenke, M. Human enteric defensin genes : chromosomal map position and a model for possible evolutionary relationships. Genomics 31 : 95-106 (1996).

Bonfield J. K., Smith K. F. & Staden R. A new DNA sequence assembly program. Nucleic Acids Res. 23 : 4992-9 (1995).

Buckholz R.G. Yeast Systems for the Expression of Heterologous Gene Products. Curr. Op. Biotechnology 4: 538-542 (1993).

Carter B.J. Adeno-Associated virus vectors. Curr. Op. Biotechnology 3: 533-539 (1993).

Cherif D., Julier C., Delattre O., Derré J., Lathrop G.M., & Berger R. : Simultaneous localization of cosmids and chromosome R-banding by fluorescence microscopy - Applications to regional mapping of chromosome 11. Proc.Natl.Acad.Sci. USA. 87 : 6639-6643 (1990).

Chumakov I., Rigault P., Guillou S., Ougcn P., Biliault A., Guasconi G., Gervy P., Le Gall I., Soularue P., Grinas P. et al. Continuum of overlapping cioncs spanning the entire human chromosome 21q. Nature 359: 380-386 (1992).

Chumakov I.M., Rignault P., Le Gall 1. et al. A YAC contig map of the human genome. Nature 377 supplt : 175-183 (1995).

Compton J. Nucleic Acid Sequence-Based Amplification. Nature 350: 91-92 (1991).

Danos O., Moullier P. & Heard J.M. Réimplantation de cellules génétiquement modifiées dans des néo-organes vascularisés. Médecine/Sciences 9:62-64 (1993).

Edwards C.P. et Aruffo A. Current applications of COS cell based transient expression systems. Curr. Op. Biotechnology 4: 558-563 (1993).

Epstein A. : Les vecteurs herpétiques pour le transfert de gènes - Médecine/Sciences 8: 902-911 (1992).

Ganz T. & Lehrer R.I. Defensins. Curr. Op. Immunology. 6 : 584-9 (1994).

Ganz T. & Lehrer R.I. Defensins. Pharmac. Ther. Vol. 66 : 191-205 (1995).

George D. G., Barker W. C., Mewes H. W, Pfeiffer F. & Tsugita A. The PIR-International Protein Sequence Database. Nucleic Acids Res. 24: 17-20 (1996).

Guatelli J.C. et al. Isothermal in vitro amplification of nucleic acids by a multienzyme reaction modeled after retroviral replication. Proc. Natl. Acad. Sci. USA 87: 1874-1878 (1990).

Hillier L. & Green P. OSP : a computer program for choosing PCR and DNA sequencing primers. PCR Methods Appl. 1: 124-8 (1991).

Ichikawa, T., Nihei, N., Kuramochi, H., Kawana, Y., Killary, A.M., Rinker-Schaeffer, C.W., Barrett, J.C., Isaacs, J.T., Kugoh, H., Oshimura, M. & Shimazaki, J. Metastasis suppressor genes for prostate cancer. Prostate Suppl. 6 : 31-35 (1996).

Kagan, B.L., Ganz, T. & Lehrer, R.I. Defensins : a family of antimicrobial and cytotoxic peptides. Toxicology 87 : 131-149 (1994).

Landegren U., Kaiser R., Sandcrs J. & Hood L.A ligase-mediated gene detection technique. Science241: 1077-1080 (1988).

Lehrer & Ganz. Defensins : endogenous antibiotic peptides from human leukocytes. Ciba Found. Sympo. 171 : 276-290 ( 1992).

Luckow V.A. Baculovirus systems for the expression of human gene products. Curr. Op. Biotechnology 4: 564-572 (1993).

Mallow, E.B., Harris, A., Salzman, N., Russel, J.P., DeBerardinis, R.J., Ruchelli, E., & Bevins, C.L. Human enteric defensins. Gene structure and developmental expression. J. Biol. Chem. 271 (8) : 4038-4045 (1996).

Martin, E., Ganz, T. & Lehrer, R.I. Defensins and othcr endogenous peptide antibiotics of vertebrates. J. Leukocyte Biol. 58 : 128-136 (1995).

Olins P.O. et Lee S.C. Recent advances in heterologous gene expression in E. coli. Curr. Op. Biotechnology 4: 520-525 (1993).

Perricaudet M., Stratford-Perricaudet L., & Briand P. : La thérapie génique par adénovirus - La Recherche 23: 471-473 (1992).

Rodriguez-Tome P., Stoehr P. J., Cameron G. N., & Flores T. P. The European Bioinformatics Institute (EBI) databases. Nucleic Acids Res. 24: 6-12 (1996).

Samiotaki M., Kwiatkowksi M., Parik J., & Landegren U. Dual-color detection of DNA sequence variants through ligase-mediated analysis. Genomics 20: 238-242 (1994).

Sparkes, R.S., Kronenberg, M., Heinzmann, C., Daher, K.A., Klisak, I., Ganz, T. & Mohandas, T. Assignment of defensin gene(s) to human chromosome 8p23. Genomics 5 (2) : 240-4 (1989).

Stewart, J.M. et Yound, J.D. Solid Phase Peptides Synthesis. Pierce Chem. Company, Rockford, 111, 2ème éd., (1984).

Stoneking M., Hedgecock D., Higuchi R.G., Vigilant L., & Erlich H.A. Population variation of human DNA control region sequences by enzymatic amplification and sequence-specific oligonucleotide probes. Am. J. Hum. Genet. 48: 370-382 (1991).

Sundareshan, T.S. & Augustus, M. Cytogenetics of non-small cell lung cancer : simple technique for obtaining high quality chromosomes by fine needle aspirate cultures. Cancer Genet. Cytogenet. 91 (1) : 53-60 (1996).

Syvänen A.C., Aalto-Setala K., Harju L., Kontula K. & Soderlund H. A primer-guided nucleotide incorporation assay in the genotyping of Apo E. Genomics 8 : 684-692 (1990).

Temin H.M. : Retrovirus vectors for gene transfer. In Kucherlapati R., ed. Gene Transfer, New York, Plenum Press, 149-187 (1986).

Walker G.T., Fraiser M.S., Schram J.L., Little M.C., Nadeau J.G., & Malinowski D.P. Strand displacement amplification : an isothermal in vitro DNA amplification technique. Nucleic Acids Res. 20: 1691-1696 (1992).

White, S.H., Wimley, W.C. & Selsted, M.E. Structure, function, and membrane integration of defensins. Curr. Op. Structural Biology. 5 : 521-527 (1995).

Wu D.Y., Ugozzoli L., Pal B.K. & Wallace R.B. Allele-specific amplification of b-globin genomic DNA for diagnosis of sickle cell anemia. Proc. Natl. Acad. Sci. USA 86 : 2757-2760 (1989).

Yaremko, M.L., Wasylyshyn, M.L., Paulus, K.L., Michelassi, F. & Westbrook, C.A. Deletion mapping reveals two regions of chromosome 8 allele loss in colorectal carcinomas. Genes Chromosomes Cancer. 10 (1) : 1-6 (1994).

## Revendications

1. Polypeptide isolé choisi parmi les polypeptides suivants :
a) polypeptide dont la séquence d'acides aminés est la séquence SEQ ID No. 6 ;
b) polypeptide présentant au minimum 80 % d'identité avec le polypeptide dont la séquence d'acides aminés est la séquence SEQ ID No. 6 ;
c) polypeptide dont la séquence d'acides aminés est la séquence d'acides aminés d'un fragment biologiquement actif d'au moins 10 acides aminés consécutifs du polypeptide tel que défini en a), ledit fragment ayant au moins une activité choisie parmi les activités suivantes :
• reconnaissance par un anticorps spécifique du polypeptide défini en a) ;
• activité antibiotique ;
• activité cytotoxique ; ou
• activité antitumorale ; et
d) polypeptide comprenant au moins un polypeptide tel que défini en a), b), ou c).

2. Polypeptide selon la revendication 1, **caractérisé en ce que** ledit fragment comprend au moins 15 acides aminés consécutifs.

3. Polypeptide selon la revendication 1, **caractérisé en ce que** la séquence d'acides aminés dudit polypeptide est la séquence SEQ ID No. 3.

4. Polypeptide selon l'une des revendications 1 à 3, **caractérisé en ce qu'**il est obtenu par synthèse chimique.

5. Acide nucléique isolé codant pour un polypeptide selon l'une des revendications 1 à 3.

6. Acide nucléique selon la revendication 5, choisi parmi les acides nucléiques suivants :
a) acide nucléique de séquence SEQ ID No. 1 ;
b) acide nucléique de séquence SEQ ID No. 2 ;
c) acide nucléique présentant 80 % d'identité par rapport aux acides nucléiques selon a) ou b) ; et
d) fragment nucléique de la séquence SEQ ID No. 1 comprenant au moins un exon de séquence nt 1836 à 1874, nt 3394 à 3577 ou nt 4164 à 4379 de la séquence SEQ ID No. 1.

7. Vecteur de clonage ou d'expression dans une cellule hôte appropriée d'une séquence nucléotidique, **caractérisé en ce qu'**il comporte une séquence selon l'une des revendications 5 ou 6.

8. Vecteur selon la revendication 7, **caractérisé en ce qu'**il comporte les éléments assurant l'expression de ladite séquence dans ladite cellule hôte.

9. Cellule transformée par un vecteur selon l'une des revendications 7 et 8.

10. Cellule selon la revendication 9, **caractérisée en ce qu'**il s'agit d'une cellule procaryote.

11. Cellule selon la revendication 9, **caractérisée en ce qu'**il s'agit d'une cellule eucaryote.

12. Procédé de production d'un polypeptide selon l'une des revendications 1 à 3, **caractérisé en ce qu'**on cultive une cellule selon l'une des revendications 8 à 10 et **en ce que** l'on récupère le polypeptide produit.

13. Anticorps monoclonal ou polyclonal ou un de leurs fragments, anticorps chimériques, **caractérisé en ce qu'**il est capable de reconnaître spécifiquement un polypeptide selon l'une des revendications 1 à 4.

14. Anticorps selon la revendication 13, **caractérisé en ce qu'**il est marqué.

15. Sonde ou amorce oligonucléotidique, **caractérisée en ce qu'**elle est constituée d'un acide nucléique selon l'une des revendications 5 et 6.

16. Sonde selon la revendication 15, **caractérisée en ce qu'**elle est marquée.

17. Polypeptide selon l'une des revendications 1 à 4, pour utilisation comme agent antimicrobien, antiparasitaire, et/ou antiviral.

18. Polypeptide selon l'une des revendications 1 à 4, pour utilisation comme agent cytotoxique.

19. Polypeptide selon la revendication 18, **caractérisé en ce que** ledit agent cytotoxique est à visée anticancéreuse.

20. Polypeptide selon l'une des revendications 1 à 4, pour utilisation comme agent de modulation des processus de l'inflammation, de réparation tissulaire et de régulation endocrine.

21. Polypeptide selon la revendication 20, **caractérisé en ce que** ladite modulation est corticostatique.

22. Composition pharmaceutique comprenant un polypeptide selon l'une des revendications 1 à 4.

23. Composition pharmaceutique selon la revendication 22 pour usage topique externe, **caractérisée en ce qu'**elle comporte au moins un polypeptide selon l'une des revendications 1 à 4.

24. Composition cosmétique pour usage topique externe comprenant un polypeptide selon l'une des revendications 1 à 4.

25. Composition selon l'une des revendications 23 ou 24, **caractérisée en ce qu'**il s'agit d'une composition utilisée comme agent antiseptique.

26. Composition pharmaceutique comprenant un vecteur selon l'une des revendications 7 et 8, capable d'exprimer *in vivo* un polypeptide selon l'une des revendications 1 à 3.

27. Composition pharmaceutique comprenant une cellule transformée selon l'une des revendications 9 à 11, capable d'exprimer *in vivo* un polypeptide selon l'une des revendications 1 à 3.

28. Composition pharmaceutique selon l'une des revendications 22, 23, 26 et 27, **caractérisée en ce qu'**elle comprend un véhicule pharmaceutiquement acceptable.

29. Composition pharmaceutique selon l'une des revendications 22, 23, et 26 à 28, destinée à la prévention et/ou au traitement des infections microbiennes, parasitaires, ou virales.

30. Composition pharmaceutique selon la revendication 29, **caractérisée en ce que** les infections microbiennes ou parasitaires sont des infections d'origines bactériennes, de bactéries Gram-positives ou Gram-négatives, mycobactériennes, fongiques, ou liées à des spirochètes.

31. Composition pharmaceutique selon la revendication 29, **caractérisée en ce que** les infections virales sont des infections liées à des virus à enveloppe.

32. Composition pharmaceutique selon la revendication 31, **caractérisée en ce que** lesdits virus à enveloppe sont les virus HSV et HIV.

33. Composition pharmaceutique selon l'une des revendications 22, 23 et 26 à 28, destinée à la prévention et/ou au traitement de cancers.

34. Composition pharmaceutique selon la revendication 33, **caractérisée en ce que** lesdits cancers sont des mélanomes.

35. Composition pharmaceutique selon la revendication 34, **caractérisée en ce que** le cancer est le cancer du foie, de la prostate, du poumon non à petites cellules ou le carcinome colorectal.

36. Composition pharmaceutique selon l'une des revendications 22, 23 et 26 à 28, destinée à augmenter les défenses immunitaires, à augmenter les défenses immunitaires en cas d'immunodéficience acquise ou à prévenir l'immunodéficience.

37. Composition pharmaceutique selon la revendication 36, **caractérisée en ce qu'**elle est destinée à traiter du psoriasis.

38. Composition pharmaceutique selon l'une des revendications 22, 23 et 26 à 28, destinée à moduler les processus inflammatoires.

39. Composition pharmaceutique selon la revendication 38, **caractérisée en ce qu'**elle est destinée à moduler les processus inflammatoires des maladies à inflammation chronique.

40. Méthode de mise en évidence d'une expression anormale de la protéine de séquence SEQ ID No. 3 dans un échantillon biologique préalablement prélevé chez un patient souffrant d'inflammation, d'immunodéficience, de prédispositions à des affections de type cancer et/ou d'infections dues à des micro-organismes ou liées à un déficit immunitaire ou phénomène inflammatoire, **caractérisée en ce qu'**elle comprend les étapes suivantes :
a) le dosage d'un polypeptide selon l'une des revendications 1 à 3 dudit échantillon biologique ; et
b) la comparaison du résultat dudit dosage obtenu avec la quantité dudit polypeptide normalement présente dans un échantillon biologique équivalent.

41. Méthode de mise en évidence d'une expression anormale de la protéine de séquence SEQ ID No. 3 dans un échantillon biologique préalablement prélevé chez un patient souffrant d'inflammation, d'immunodéficience, de prédispositions à des affections de type cancer et/ou d'infections dues à des micro-organismes ou liées à un déficit immunitaire ou phénomène inflammatoire, **caractérisée en ce qu'**elle comprend les étapes suivantes :
c) le dosage d'un acide nucléique selon l'une des revendications 5 et 6 dudit échantillon biologique ; et
d) la comparaison du résultat dudit dosage obtenu avec la quantité dudit acide nucléique normalement présente dans un échantillon biologique équivalent.

42. Méthode selon la revendication 40, **caractérisée en ce qu'**elle met en oeuvre un anticorps selon l'une des revendications 13 et 14.

43. Méthode selon la revendication 41, **caractérisée en ce qu'**elle met en oeuvre une sonde et/ou une amorce oligonucléotidique selon l'une des revendications 15 et 16.

44. Kit ou nécessaire de diagnostic pour la détermination d'une infection microbienne ou parasitaire, d'une inflammation, d'une immunodéficience et/ou de prédisposition à des affections de type cancer, **caractérisé en ce qu'**il comprend un anticorps selon l'une des revendications 13 et 14.

45. Kit ou nécessaire de diagnostic pour la détermination d'une infection microbienne ou parasitaire, d'une inflammation, d'une immunodéficience et/ou de prédisposition à des affections de type cancer, **caractérisé en ce qu'**il comprend une sonde et/ou une amorce selon l'une des revendications 15 et 16.

46. Utilisation d'un polypeptide selon l'une des revendications 1 à 4, comme pesticide.

47. Utilisation selon la revendication 46, **caractérisée en ce que** ledit pesticide est utilisé pour la culture de végétaux d'intérêt industriel.

## Patentansprüche

1. Isoliertes Polypeptid, ausgewählt aus den folgenden Polypeptiden:
a) Polypeptid, dessen Aminosäure-Sequenz die Sequenz SEQ ID Nr. 6 ist;
b) Polypeptid, das zu mindestens 80 % identisch ist mit dem Polypeptid, dessen Aminosäure-Sequenz die Sequenz SEQ ID Nr. 6 ist;
c) Polypeptid, dessen Aminosäure-Sequenz die Aminosäure-Sequenz eines biologisch aktiven Fragments aus mindestens 10 aufeinanderfolgenden Aminosäuren des Polypeptids ist, wie es unter (a) definiert ist, wobei das genannte Fragment mindestens eine Aktivität, ausgewählt unter den folgenden Aktivitäten, aufweist:
• Erkennung des unter (a) definierten Polypeptids durch einen spezifischen Antikörper;
• antibiotische Aktivität;
• zytotoxische Aktivität; oder
• Antitumor-Aktivität; und
d) Polypeptid, das mindestens ein Polypeptid umfasst, wie es unter (a), (b) oder (c) definiert ist.

2. Polypeptid nach Anspruch 1, **dadurch gekennzeichnet, dass** das genannte Fragment mindestens 15 aufeinanderfolgende Aminosäuren umfasst.

3. Polypeptid nach Anspruch 1, **dadurch gekennzeichnet, dass** die Aminosäure-Sequenz des genannten Polypeptids die Sequenz SEQ ID Nr. 3 ist.

4. Polypeptid nach einem der Ansprüche 1 bis 3, **dadurch gekennzeichnet, dass** es durch chemische Synthese erhalten worden ist.

5. Isolierte Nucleinsäure, die für ein Polypeptid nach einem der Ansprüche 1 bis 3 codiert.

6. Nucleinsäure nach Anspruch 5, die ausgewählt ist unter den folgenden Nucleinsäuren:
a) Nucleinsäure mit der Sequenz SEQ ID Nr. 1;
b) Nucleinsäure mit der Sequenz SEQ ID Nr. 2;
c) Nucleinsäure, die zu 80 % identisch ist mit den Nucleinsäuren gemäß (a) oder (b); und
d) Nucleinsäure-Fragment mit der Sequenz SEQ ID Nr. 1, das mindestens ein Sequenz-Exon nt 1836 bis 1874, nt 3394 bis 3577 oder nt 4164 bis 4379 der Sequenz SEQ ID Nr. 1 umfasst.

7. Vektor zum Klonieren oder Exprimieren einer Nucleotid-Sequenz in einer geeigneten Wirtszelle, **dadurch gekennzeichnet, dass** er eine Sequenz nach einem der Ansprüche 5 oder 6 umfasst.

8. Vektor nach Anspruch 7, **dadurch gekennzeichnet, dass** er die Elemente umfasst, die eine Expression der genannten Sequenz in der genannten Wirtszelle gewährleisten.

9. Zelle, die durch einen Vektor nach einem der Ansprüche 7 und 8 transformiert worden ist.

10. Zelle nach Anspruch 9, **dadurch gekennzeichnet, dass** es sich dabei um eine Prokaryontenzelle handelt.

11. Zelle nach Anspruch 9, **dadurch gekennzeichnet, dass** es sich dabei um eine Eukaryontenzellen handelt.

12. Verfahren zur Herstellung eines Polypeptids nach einem der Ansprüche 1 bis 3, **dadurch gekennzeichnet, dass** man eine Zelle nach einem der Ansprüche 8 bis 10 kultiviert und das gebildete Polypeptid daraus gewinnt.

13. Monoklonaler oder polyklonaler Antikörper oder eines ihrer Fragmente, Chimären-Antikörper, **dadurch gekennzeichnet, dass** er (es) in der Lage ist, ein Polypeptid nach einem der Ansprüche 1 bis 4 spezifisch zu erkennen.

14. Antikörper nach Anspruch 13, **dadurch gekennzeichnet, dass** er markiert ist.

15. Oligonucleotid-Sonde oder -Initiator, **dadurch gekennzeichnet, dass** sie (er) aus einer Nucleinsäure nach einem der Ansprüche 5 und 6 besteht.

16. Sonde nach Anspruch 15, **dadurch gekennzeichnet, dass** sie markiert ist.

17. Polypeptid nach einem der Ansprüche 1 bis 4 für die Verwendung als antimikrobielles, antiparasitäres und/oder antivirales Agens.

18. Polypeptid nach einem der Ansprüche 1 bis 4 für die Verwendung als zytotoxisches Agens.

19. Polypeptid nach Anspruch 18, **dadurch gekennzeichnet, dass** das genannte zytotoxische Agens als Antikrebsmittel verwendbar ist.

20. Polypeptid nach einem der Ansprüche 1 bis 4 für die Verwendung als Agens zur Modulierung von Entzündungs-, Gewebsreparatur- und endokrinen Regulations-Prozessen.

21. Polypeptid nach Anspruch 20, **dadurch gekennzeichnet, dass** die genannte Modulation eine corticostatische Modulation ist.

22. Pharmazeutische Zusammensetzung, die ein Peptid nach einem der Ansprüche 1 bis 4 umfasst.

23. Pharmazeutische Zusammensetzung nach Anspruch 22 für die äußere topische Anwendung, **dadurch gekennzeichnet, dass** sie mindestens ein Peptid nach einem der Ansprüche 1 bis 4 umfasst.

24. Kosmetische Zusammensetzung für die äußere topische Anwendung, die ein Polypeptid nach einem der Ansprüche 1 bis 4 umfasst.

25. Zusammensetzung nach einem der Ansprüche 23 oder 24, **dadurch gekennzeichnet, dass** es sich dabei um eine Zusammensetzung handelt, die als antiseptisches Agens verwendet wird.

26. Pharmazeutische Zusammensetzung, die einen Vektor nach einem der Ansprüche 7 und 8 umfasst, der in der Lage ist, ein Polypeptid nach einem der Ansprüche 1 bis 3 in vivo zu exprimieren.

27. Pharmazeutische Zusammensetzung, die eine transformierte Zelle nach einem der Ansprüche 9 bis 11 umfasst, die in der Lage ist, ein Polypeptid nach einem der Ansprüche 1 bis 3 in vivo zu exprimieren.

28. Pharmazeutische Zusammensetzung nach einem der Ansprüche 22, 23, 26 und 27, **dadurch gekennzeichnet, dass** sie ein pharmazeutisch akzeptables Vehiculum umfasst.

29. Pharmazeutische Zusammensetzung nach einem der Ansprüche 22, 23 und 26 bis 28, die für die Prävention und/oder Behandlung von Mikroben-, Parasiten- oder Viren-Infektionen bestimmt ist.

30. Pharmazeutische Zusammensetzung nach Anspruch 29, **dadurch gekennzeichnet, dass** die Mikroben- oder Parasiten-Infektionen Infektionen bakteriellen Ursprungs, Infektionen von Gram-positiven oder Gram-negativen Bakterien, Mycobakterien, Fungi sind oder mit Spirochäten in Verbindung stehen.

31. Pharmazeutische Zusammensetzung nach Anspruch 29, **dadurch gekennzeichnet, dass** die viralen Infektionen Infektionen sind, die mit Hüllen-Viren in Verbindung stehen.

32. Pharmazeutische Zusammensetzung nach Anspruch 31, **dadurch gekennzeichnet, dass** die genannten Hüllen-Viren die Viren HSV und HIV sind.

33. Pharmazeutische Zusammensetzung nach einem der Ansprüche 22, 23 und 26 bis 28, die für die Prävention und/oder Behandlung von Krebsen bestimmt sind.

34. Pharmazeutische Zusammensetzung nach Anspruch 33, **dadurch gekennzeichnet, dass** die genannten Krebse Melanome sind.

35. Pharmazeutische Zusammensetzung nach Anspruch 34, **dadurch gekennzeichnet, dass** es sich bei dem Krebs um Leberkrebs, Prostatakrebs, Lungenkrebs mit kleinen Zellen oder um das Colorectal-Carcinom handelt.

36. Pharmazeutische Zusammensetzung nach einem der Ansprüche 22, 23 und 26 bis 28, die zur Erhöhung der Immunabwehr, zur Erhöhung der Immunabwehr im Falle einer erworbenen Immundefizienz oder zur Prävention der Immundefizienz bestimmt ist.

37. Pharmazeutische Zusammensetzung nach Anspruch 36, **dadurch gekennzeichnet, dass** sie zur Behandlung der Psoriasis bestimmt ist.

38. Pharmazeutische Zusammensetzung nach einem der Ansprüche 22, 23 und 26 bis 28, dass sie zur Modulation der entzündlichen Prozesse bestimmt ist.

39. Pharmazeutische Zusammensetzung nach Anspruch 38, **dadurch gekennzeichnet, dass** sie dazu bestimmt ist, die Entzündungsprozesse der Erkrankungen mit chronischer Entzündung zu modulieren.

40. Verfahren zum Nachweis einer anormalen Expression des Proteins mit der Sequenz SEQ ID Nr. 3 in einer vorher entnommenen biologischen Probe eines Patienten, der leidet unter einer Entzündung, einer Immundefizienz, Prädispositionen für Erkrankungen vom Krebs-Typ und/oder Infektionen durch Mikroorganismen oder solchen, die mit einem Immundefizit oder einem Entzündungsphänomen in Verbindung stehen, **dadurch gekennzeichnet, dass** es die folgenden Stufen umfasst:
a) Bestimmung eines Polypeptids nach einem der Ansprüche 1 bis 3 in der biologischen Probe; und
b) Vergleich des erhaltenen Ergebnisses der Bestimmung mit der Menge des Polypeptids, die normalerweise in einer äquivalenten biologischen Probe vorhanden ist.

41. Verfahren zum Nachweis einer anormalen Expression des Proteins mit der Sequenz SEQ ID Nr. 3 in einer vorher entnommenen biologischen Probe eines Patienten, der leidet unter einer Entzündung, einem Immundefizit, unter Prädispositionen für Erkrankungen vom Krebs-Typ und/oder Infektionen, die auf Mikroorganismen zurückzuführen sind oder mit einem Immundefizit oder einem Entzündungsphänomen in Verbindung stehen, **dadurch gekennzeichnet, dass** es die folgenden Stufen umfasst:
c) Bestimmung einer Nucleinsäure nach einem der Ansprüche 5 und 6 in der biologischen Probe; und
d) Vergleich des erhaltenen Ergebnisses der Bestimmung mit der Menge der Nucleinsäure, die normalerweise in einer äquivalenten biologischen Probe vorhanden ist.

42. Verfahren nach Anspruch 40, **dadurch gekennzeichnet, dass** man einen Antikörper nach einem der Ansprüche 13 und 14 einsetzt.

43. Verfahren nach Anspruch 41, **dadurch gekennzeichnet, dass** man eine Oligonucleotid-Sonde und/oder einen Oligonucleotid-Initiator nach einem der Ansprüche 15 und 16 einsetzt.

44. Diagnostisches Kit oder diagnostisches Gerät zur Bestimmung einer Mikroben- oder Parasiten-Infektion, einer Entzündung, eines Immundefizits und/oder einer Prädisposition für Erkrankungen vom Krebs-Typ, **dadurch gekennzeichnet, dass** er (es) einen Antikörper nach einem der Ansprüche 13 und 14 umfasst.

45. Diagnostisches Kit oder diagnostisches Gerät zur Bestimmung einer Mikroben- oder Parasiten-Infektion, einer Entzündung, eines Immundefizits und/oder einer Prädisposition für Erkrankungen vom Krebs-Typ, **dadurch gekennzeichnet, dass** er (es) eine Sonde und/oder einen Initiator nach einem der Ansprüche 15 und 16 umfasst.

46. Verwendung eines Polypeptids nach einem der Ansprüche 1 bis 4 als Pestizid.

47. Verwendung nach Anspruch 46, **dadurch gekennzeichnet, dass** das genannte Pestizid zur Kultivierung von industriell verwertbaren Pflanzen eingesetzt wird.

## Claims

1. Isolated polypeptide selected from the following polypeptides:
a) polypeptide for which the sequence of amino acids is the sequence SEQ ID No 6;
b) polypeptide with a minimum of 80% identity with the polypeptide for which the sequence of amino acids is the sequence SEQ ID No 6;
c) polypeptide for which the sequence of amino acids is the sequence of amino acids for a biologically active fragment with at least 10 consecutive amino acids of the polypeptide as defined in a), this fragment having at least one activity selected from the following activities:
• recognition by a specific antibody of the polypeptide defined in a);
• antibiotic activity;
• cytotoxic activity; or
• anti-tumor activity; and
d) polypeptide comprising at least one polypeptide as defined in a), b) or c).

2. Polypeptide according to claim 1, **characterised by** the fact that this fragment comprises at least 15 consecutive amino acids.

3. Polypeptide according to claim 1, **characterised by** the fact that the sequence of amino acids of this polypeptide is the sequence SEQ ID No 3.

4. Polypeptide according to one of claims 1 to 3, **characterised by** the fact that it is obtained by chemical synthesis.

5. Isolated coding nucleic acid for a polypeptide according to one of claims 1 to 3.

6. Nucleic acid according to claim 5 selected from the following nucleic acids:
a) nucleic acid of sequence SEQ ID No 1;
b) nucleic acid of sequence SEQ ID No 2;
c) nucleic acid with 80% identity in relation to the nucleic acids according to a) or b); and
d) nucleic fragment of the sequence SEQ ID No 1 comprising at least one exon of sequence nt 1836 to 1874, nt 3394 to 3577 or nt 4164 to 4379 of the sequence SEQ ID No 1.

7. Expression or cloning vector in an appropriate host cell of a nucleotidic sequence, **characterised by** the fact that it comprises a sequence according to one of claims 5 or 6.

8. Vector according to claim 7, **characterised by** the fact that it comprises elements providing expression of this sequence in this host cell.

9. Cell transformed by a vector according to one of claims 7 and 8.

10. Cell according to claim 9, **characterised by** the fact that it is a procaryotic cell.

11. Cell according to claim 9, **characterised by** the fact that it is a eucaryotic cell.

12. Process for production of a polypeptide according to one of claims 1 to 3, **characterised by** the fact that a cell is cultivated according to one of claims 8 to 10 and that the polypeptide produced is recovered.

13. Monoclonal or polyclonal antibody or one of their fragments and chimeric antibody, **characterised by** the fact that it is capable of recognising specifically a polypeptide according to one of claims 1 to 4.

14. Antibody according to claim 13, **characterised by** the fact that it is labelled.

15. Probe or oligonucleotidic primer, **characterised by** the fact that it is made up of a nucleic acid according to one of claims 5 and 6.

16. Probe according to claim 15, **characterised by** the fact that it is labelled.

17. Polypeptide according to one of claims 1 to 4 for use as antimicrobial, antiparasitic and/or antiviral agent.

18. Polypeptide according to one of claims 1 to 4 for use as cytotoxic agent.

19. Polypeptide according to claim 18, **characterised by** the fact that this cytotoxic agent has an anticancer aim.

20. Polypeptide according to one of claims I to 4 for use as modulation agent for inflammation, tissue repair and endocrine regulation processes.

21. Polypeptide according to claim 20, **characterised by** the fact that this modulation is corticostatic.

22. Pharmaceutical composition comprising a polypeptide according to one of claims 1 to 4.

23. Pharmaceutical composition according to claim 22 for external local use, **characterised by** the fact that it comprises at least one polypeptide according to one of claims 1 to 4.

24. Cosmetic composition for external local use comprising a polypeptide according to one of claims 1 to 4.

25. Composition according to one of claims 23 or 24, **characterised by** the fact that it is a composition used as antiseptic agent.

26. Pharmaceutical composition comprising a vector according to one of claims 7 and 8 capable of expressing *in vivo* a polypeptide according to one of claims 1 to 3.

27. Pharmaceutical composition comprising a cell transformed according to one of claims 9 to 11, capable of expressing *in vivo* a polypeptide according to one of claims 1 to 3.

28. Pharmaceutical composition according to one of claims 22, 23, 26 and 27, **characterised by** the fact that it comprises a pharmaceutically acceptable vehicle.

29. Pharmaceutical composition according to one of claims 22, 23 and 26 to 28, intended for the prevention and/or treatment of microbial, parasitic or viral infections.

30. Pharmaceutical composition according to claim 29, **characterised by** the fact that the microbial or parasitic infections are infections of bacterial origin, Grampositive or Gramnegative bacteria, mycobacterial and fungal or connected with spirochetes.

31. Pharmaceutical composition according to claim 29, **characterised by** the fact that the viral infections are infections connected to membrane viruses.

32. Pharmaceutical composition according to claim 31, **characterised by** the fact that these membrane viruses are HSV and HIV viruses.

33. Pharmaceutical composition according to one of claims 22, 23 and 26 to 28 intended for the prevention and/or treatment of cancers.

34. Pharmaceutical composition according to claim 33, **characterised by** the fact that these cancers are melanomas.

35. Pharmaceutical composition according to claim 34, **characterised by** the fact that the cancer is cancer of the liver, prostate and lung and not small cell cancer or colorectal carcinoma.

36. Pharmaceutical composition according to one of claims 22, 23 and 26 to 28 intended to increase immune defences, to increase immune defences in case of acquired immunodeficiency or prevent immunodeficiency.

37. Pharmaceutical composition according to claim 36, **characterised by** the fact that it is intended to treat psoriasis.

38. Pharmaceutical composition according to one of claims 22, 23 and 26 to 28 intended to treat inflammatory processes.

39. Pharmaceutical composition according to claim 38, **characterised by** the fact that it is intended to modulate the inflammatory processes of diseases with chronic inflammation.

40. Method for showing an abnormal expression of the protein of sequence SEQ ID No 3 in a biological sample previously taken from a patient suffering from inflammation, immunodeficiency, predispositions to cancer type illnesses and/or infections due to microorganisms or connected to an immunodeficiency or inflammatory phenomenon, **characterised by** the fact that it comprises the following stages:
a) dosing with a polypeptide according to one of claims 1 to 3 of this biological sample; and
b) comparison of the result of this dosing obtained with the quantity of this polypeptide normally present in an equivalent biological sample.

41. Method for showing an abnormal expression of the protein of sequence SEQ ID No 3 in a biological sample previously taken from a patient suffering from inflammation, immunodeficiency, predispositions to cancer type illnesses and/or infections due to microorganisms or connected to an immunodeficiency or inflammatory phenomenon, **characterised by** the fact that it comprises the following stages:
c) dosing with a nucleic acid according to one of claims 5 and 6 of this biological sample; and
d) comparison of the result of this dosing obtained with the quantity of this nucleic acid normally present in an equivalent biological sample.

42. Method according to claim 40, **characterised by** the fact that it puts an antibody according to one of claims 13 and 14 into operation.

43. Method according to claim 41, **characterised by** the fact that it puts a probe and/or an oligonucleotidic primer according to one of claims 15 and 16 into operation.

44. Diagnostic kit for determining a microbial or parasitic infection, inflammation, immunodeficiency and/or predisposition to cancer type illnesses, **characterised by** the fact that it comprises an antibody according to one of claims 13 and 14.

45. Diagnostic kit for determining a microbial or parasitic infection, inflammation, immunodeficiency and/or predisposition to cancer type illnesses, **characterised by** the fact that it comprises a probe and/or primer according to one of claims 15 and 16.

46. Use of a polypeptide according to one of claims 1 to 4 as pesticide.

47. Use according to claim 46, **characterised by** the fact that this pesticide is used for cultivating vegetables of industrial interest.


### LISTE DE SEQUENCES

**(1) INFORMATIONS GENERALES:**
   (i) DEPOSANT:
      (A) NOM: GENSET SA
      (B) RUE: 24 RUE ROYALE
      (C) VILLE: PARIS
      (E) PAYS: FRANCE
      (F) CODE POSTAL: 75008
   (ii) TITRE DE L' INVENTION: POLYPEPTIDE DEFENSINE HUMAINE Def-X, ADN GENOMIQUE ET ADNc, COMPOSITION LES CONTENANT ET APPLICATIONS AU DIAGNOSTIC ET AU TRAITEMENT THERAPEUTIQUE
   (iii) NOMBRE DE SEQUENCES: 6
   (iv) FORME DECHIFFRABLE PAR ORDINATEUR:
      (A) TYPE DE SUPPORT: Floppy disk
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
**(2) INFORMATIONS POUR LA SEQ ID NO: 1:**
   (i) CARACTERISTIQUES DE LA SEQUENCE:
      (A) LONGUEUR: 4415 PAIRES DE BASE
      (B) TYPE: NUCLEOTIDE
      (C) NOMBRE DE BRINS: DOUBLE
      (D) CONFIGURATION: LINEAIRE
   (ii) TYPE DE MOLECULE: ADN
   (vi) ORIGINE:
      (A) ORGANISME: Homo sapiens
   (ix) CARACTERISTIQUE:
      (A) NOM/CLE: Exon 1

      (B) EMPLACEMENT: 1836..1874
   (ix) CARACTERISTIQUE:
      (A) NOM/CLE: Exon 2
      (B) EMPLACEMENT: 3394..3577
   (ix) CARACTERISTIQUE:
      (A) NOM/CLE: Exon 3
      (B) EMPLACEMENT: 4161..4380
   (ix) CARACTERISTIQUE:
      (A) NOM/CLE: start CDS
      (B) EMPLACEMENT: 3406..3408
   (ix) CARACTERISTIQUE:
      (A) NOM/CLE: stop CDS
      (B) EMPLACEMENT: 4276..4278
   (ix) CARACTERISTIQUE:
      (A) NOM/CLE: site de polyAdenylation

      (B) EMPLACEMENT: 4374..4379
   (xi) SEQUENCE DESCRIPTION: SEQ ID NO: 1:
   ACACCATTTG TCTTCATGTA ACCCCATTAG CTATACCCTC TAGTGCAAGG AAACCATAGG 60
   GCCTAGGTCA CACCATGAGG CTGCNCTTAC AAGTTATGCA AAAACTATGG ACTTGGGAGA 120
   CCTGTGCGTA ACAACATCAC ACNCCAAATT TAACCAGCTC TCCCCATAAC AGCACGCTCA 180
   TGTGTTACTG AGGAAATGCC TGTGGATTGG AGTGTGTTCT GTGTGCAGGA GGCTGGTCCA 240
   GGTTTCACTT CTGCAGGACA CTGGACGTTT CCCAAAACCA GCAGACTTTC CCCACGTGCA 300
   CACACACCCC TTCTCATTTT GCCTCTACAT CCATATCCAC TGGGCCCTTC AGGCACCTAC 360
   TAATGCCCTA GAACCTAAAA CCATCATCTG GGGCCCAGTT CCCTGAATGG CCCTAATCTC 420
   TTCCTCTGCT GGAATGAGTC CAGTGCCCAC TTCCTCCAAC GGTGAAATTG CTGGGCTGCT 480
   ACAGATCAGG AACTCACTGC TTCCTCATAG GGGCAGCCGA CTTCACTGCT CTGCAACAGC 540
   GACCACCCCT AGCGAGGCTT GAGATGCCTC TTGCCTCCTT AAGACTGAGG GAGACGCTTC 600
   AGCTCTCACT CCACTGCCCC AAGTCCTCCA CAGCGCGGTG CCTGCTGCCT TCACACAGAG 660
   CTGCAGGGGN AGGTCCTGTG TATCCGGCCT GCTGGACCAG CGCTGTGCAC AACCCTCCCA 720
   TGGCAACAGT GGCTGCCCGG CCTGCACACT GGGCTTGGCA ACCTCGCTGT AGGTATTTAT 780
   TCCCTCAGGA GTGACTGCAT TCTTTTCCCA TTTCCAGAAA ACTGATGCCA TTTACCTCAC 840
   TATGAGGAGG AGGAGGAGGA GGAGGGTGGA GAGTGGTACA TTTTAAAATG TGCACTATTC 900
   TCCCTAGGAC TCCCCCTCAA ATAACCCAGG AGGGACCATA CCAGCTCATT CCTGTGTATC 960
   CCAAGCATAN GAGTAATCAT CCCACTCATG CTGAGTGTAT GGTGGCCATT AAGCCTGCCC 1020
   TGAACTGGCT TTAGAACAAG GTGTTTGAGC ACACAGCACC GTCTTGCTGC CACCTTGGCC 1080
   CCCTCCCTTG TGAGACCTCT GAGACACATT NAGGTCTCAC CTAAAAATCT CAGGATTTCT 1140
   AGGCCCAAAN CGGTCCTAAA AAATTGTTCA GTCTGAACTC TCTAAGGTCA AGAGAAGAGG 1200
   TGGTTGCTCC CTCTAAGAAA CCACATGTTG CATGTACATC CTTAATTCCG GAAAGTCCAA 1260
   CAAACCTGCC CTGCTTAGCA ACACAAGCCG AGGTGGTACT CCTCTCACCC GGGCATTCTC 1320
   CAACACACCT GTTTGTCCAA ACAGCTTTGA TTTGTTTTTA TAGTTGGACC CCAGGTTCCC 1380
   AGGAGGCTGG TTCAGGCCAT ATTCCAAATC CTCATCTGTG TGTGAGTGGC ATTCTTAGCC 1440
   TAGCCTCCTT ACAGGGTGGA TACTATGATA CACAGCCAGG CTGTCCCAGT GGCTTTCAAT 1500
   ATTCTTTTGG TCCAGATAGT TCAGCCTCAG CACCAGTGTA GGCATCACAG GGTCAATTGT 1560
   CTTAGGAGTC ATGGAGAATT CATAGTTGGT AGCTACCTGG GCCTGGCCAG GGCTGACCAT 1620
   AGACAAGGCA TCCCTCTGTG AACTCCTATT TTAATGCCAG CTTCCCAACA AATTTCTCAA 1680
   CTGCTCTTAC CAGCAGGTAT TTAAACTACT CAATAGAAAG TAACCCTGAA AATTAGGACA 1740
   CCTGTTCCCA AAAGACCCTT AAATAGGGGA AGTCCTTTCN CTGCTTGTGC ACAGCTGCTG 1800
   ATGTGGCAAC ATGAGGCCTG GGACAGGGGA CTGTCCTCTG CCCACTCTGG TAGCCTCACG 1860
   TAGCTTAACA ATCTGTCAGT AATACAATAC AAAACTTAAA CTTTCATACT GCGGTTCCAC 1920
   CCAGGAAGCT GTGTTCCCAA TCTGACCCGT GATTATGGGG CCACCTCAGA GGGNACCCAG 1980
   TGAGGGAATA TTTTGCCATC TGGGACTGTT GGTTGCTGGG GGCAGTGGCT ATGAGCTCAG 2040
   TTAATAAACT CAAGCAGTTT CCTTCCAAAC ACACATGTCC TACTTAACGT GTCCAACAGA 2100
   GATGATCATA CTCATANGCT GCTAAAACAT TANTTTTATT TTGAGAAAAG TCTATTCATG 2160
   TTCTTGGCCC ATGGAGTTTT CATTTNATTA NTTTATTTAT TTTGCAGAGA TGGAGTCTCA 2220
   CTATGTTGCT CAAGCTGGTC TCCAACTCCT GGGCTCAAGC GATCTTCCTA CTTTGGCCTT 2280
   TGAAAGCGCT GAGATTGCCT GTGTGAGCCA TCATGGGGGC TCACTGGCCC ACTGATTAAT 2340
   CAGATTAATT GTTTTTTGCT ATTGAANTTG TTTGACTTCC TTGTATATTC GGATATTTAC 2400
   CCATTCTAAC ACGTAGGGTT TGCAAATATT TTCTCTCATG TTCTGTGTTG CCTTTTCACT 2460
   CAGTTGATGG TTTCCTTTGC TGTGCAGGTG CTTTAGTGTT CAACGCAGCC CCGCTTGTCT 2520
   ATTTTCCATT TTATTGCCTG TCCCTTTGAT GTCATAGCCA AGAAATAATT GCCCAGATTA 2580
   ATGTCAAAAA GCTTTATCCC TATATATTCT TCTAGTAGTT TATGGTTTCA GATCTTATGT 2640
   TTAGGTCTTC AATCCATTGA GTTGATTTTT GTATGTGGTA TAAGAAAAAA GACCACATGT 2700
   ATACATATCT CAAATTCTAA GGTAGTATAT ATTAGACACA TACAATGTGT CTATTTACAC 2760
   ACATTGAGCT GAAAATAATA AACATATTTT TATCTTTCAA TCAACTCTAT CTCTATCTCA 2820
   CTGAACTTGT TTCACCTATA GCCTGATGAG GTTGCTGTCC TCTCTACCCC AGCTCCTATA   2880
   GGAGACTGCT CATCCCCTAA CCTCAAAAAC CCCTTCATGA GGGTGATAAT GCCCTTGAAT   2940
   CCTGCAATGA ATTAGTTCTC TACTACAGTG GAATTCAGGT CTGTTATGAG GGTCTGGATC   3000
   TCTGAAGAGA AGAGCTCTCA TTTTCAGAAA ATAAGCAGGA TTTATTCCCT GAAATTACTG   3060
   AATTAAATCA CTGTTTCGAT TACTTTTTGC AATATTAAAA GTAAATATTT AAACAGGTAA   3120
   AAACAGAAAT AATGGTAGGG TCCTTATCAT CACCGTGAAT TCCAAGCTAG CATAGACACT   3180
   AAACCTAGAG ATTCACACTA GAATGAAAGC TGGGAGAGCA GAGGAGTCTC AGAAGGATGT   3240
   GGAGGCCAAT GGACACCTGC AACCTCTCCA ACGAAATGCC TACCTCCTCT CACTGCAGCA   3300
   TCCATCTCTG AGCCTTCTCG CAGCAGAGCT ATAAATTCAG CCTGGCTCCT CCGTTCCCAC   3360
   ACATCCACTC CTGCTCTCCC TCCTCTCCTC CAGGTGACTA CAGTTATGAG GACCCTCACC   3420
   CTCCTCTCTG CCTTTCTCCT GGTGGCCCTT CAGGCCTGGG CAGAGCCGCT CCAGGCAAGA   3480
   GCTCATGAGA TGCCAGCCCA GAAGCAGCCT CCAGCAGATG ACCAGGATGT GGTCATTTAC   3540
   TTTTCAGGAG ATGACAGCTG CTCTCTTCAG GTTCCAGGTG AGAGATGCCA GCATGCAGAG   3600
   CTACAGACTA GACAGAAGGA CAGGAGACAG GCTCTGGAAT TGGATCTCAG TGGCAGATGT   3660
   CACTTAGGTG GCTATACTTA ACATCTCTGG TCCTGGATTT TCTCATATCT AAATGGAATA   3720
   GAGAACCAAA GAAATCTAAG AGATTTTTCT TTCTCCAAAA ACTTGATTCC AAGATATGAC   3780
   TGTGAAATTC ACTAGATTTA AGATATAAGG AGATGCTACC TAGTTCCTTC TGGAGCCAGA   3840
   CAAACAAGCT TAAGTATATA GGAAAATATT TCACCCTGTC TATATAGGAG GTTTTAGAAC   3900
   CTGGAGAGGA GCCTAAGAAT GTGTTCAGGT GTGTGTGTGA TGGGCAGGAA TGCAGAAAAG   3960
   TGAAGCAAAG GAGAATGAGT CTCGAATCCT GTGTGACCAG CACTGCTCTG TGTATTTATT   4020
   CCTATTGACT GAGATTGTTT GTGCTACCGG CTGTAATACA GCCAACATCA CTCATCAGCC   4080
   AACATGTGAC TTCTCCAAGA TTCCCTTTAC CACCCACTGC TGNACCCCGT ACTCAGTTTC   4140
   TGATGCTCTC TCTGGGTCCC CAGGCTCAAC AAAGGGCTTG ATCTGCCATT GCAGAGTACT   4200
   ATACTGCATT TTTGGAGAAC ATCTTGGTGG GACCTGCTTC ATCCTTGGTG AACGCTACCC   4260
   AATCTGCTGC TACTAAGCTT GCAGACTAGA GAAAAAGAGT TCATAATTTT CTTTGAGCAT   4320
   TAAAGGGAAT TGTTATTCTT ATACCTTGTC CTCGATTTCC TGTCCTCATC CCAAATAAAT   4380
   ACTTGGTAAC ATGATTTCCG GGTTTTTTTT TTTTT                               4415
**(2) INFORMATIONS POUR LA SEQ ID NO: 2:**
   (i) CARACTERISTIQUES DE LA SEQUENCE:
      (A) LONGUEUR: 453 PAIRES DE BASE
      (B) TYPE: NUCLEOTIDE
      (C) NOMBRE DE BRINS: DOUBLE
      (D) CONFIGURATION: LINEAIRE
   (ii) TYPE DE MOLECULE: ADNc
   (vi) ORIGINE:
      (A) ORGANISME: Homo sapiens
   (xi) SEQUENCE DESCRIPTION: SEQ ID NO: 2:
   CTCTGCCCAC TCTGGTAGCC TCACGTAGCT TAACAATCTG TGACTACAGT T ATG AGG        57
                                                            Met Arg
                                                             1
   ACC CTC ACC CTC CTC TCT GCC TTT CTC CTG GTG GCC CTT CAG GCC TGG        105
   Thr Leu Thr Leu Leu Ser Ala Phe Leu Leu Val Ala Leu Gln Ala Trp
            5                  10                  15
   GCA GAG CCG CTC CAG GCA AGA GCT CAT GAG ATG CCA GCC CAG AAG CAG        153
   Ala Glu Pro Leu Gln Ala Arg Ala His Glu Met Pro Ala Gln Lys Gln
       20                  25                  30
   CCT CCA GCA GAT GAC CAG GAT GTG GTC ATT TAC TTT TCA GGA GAT GAC        201
   Pro Pro Ala Asp Asp Gln Asp Val Val Ile Tyr Phe Ser Gly Asp Asp
   35                  40                  45                  50
   AGC TGC TCT CTT CAG GTT CCA GGC TCA ACA AAG GGC TTG ATC TGC CAT        249
   Ser Cys Ser Leu Gln Val Pro Gly Ser Thr Lys Gly Leu Ile Cys His
                   55                  60                  65
   TGC AGA GTA CTA TAC TGC ATT TTT GGA GAA CAT CTT GGT GGG ACC TGC        297
   Cys Arg Val Leu Tyr Cys Ile Phe Gly Glu His Leu Gly Gly Thr Cys
               70                  75                  80
   TTC ATC CTT GGT GAA CGC TAC CCA ATC TGC TGC TAC TAA GCTTGCAGAC         346
   Phe Ile Leu Gly Glu Arg Tyr Pro Ile Cys Cys Tyr  *
           85                  90                  95
   TAGAGAAAAA GAGTTCATAA TTTTCTTTGA GCATTAAAGG GAATTGTTAT TCTTATACCT      406
   TGTCCTCGAT TTCCTGTCCT CATCCCAAAT AAATACTTGG TAACATG                    453
**(2) INFORMATIONS POUR LA SEQ ID NO: 3:**
   (i) CARACTERISTIQUES DE LA SEQUENCE:
      (A) LONGUEUR: 94 ACIDES AMINES
      (B) TYPE: ACIDE AMINE
      (C) NOMBRE DE BRINS: SIMPLE
      (D) CONFIGURATION: LINEAIRE
   (ii) TYPE DE MOLECULE: PROTEINE
   (vi) ORIGINE:
      (A) ORGANISME: Homo sapiens
   (ix) CARACTERISTIQUE:
      (A) NOM/CLE: PEPTIDE SIGNAL
      (B) EMPLACEMENT: 1..19
   (ix) CARACTERISTIQUE:
      (A) NOM/CLE: REGION PRO
      (B) EMPLACEMENT: 20..63
   (ix) CARACTERISTIQUE:
      (A) NOM/CLE: PEPTIDE MATURE
      (B) EMPLACEMENT: 64..94
   (xi) SEQUENCE DESCRIPTION: SEQ ID NO: 3:
   Met Arg Thr Leu Thr Leu Leu Ser Ala Phe Leu Leu Val Ala Leu Gln
   1               5                   10                  15
   Ala Trp Ala Glu Pro Leu Gln Ala Arg Ala His Glu Met Pro Ala Gln
               20                  25                  30
   Lys Gln Pro Pro Ala Asp Asp Gln Asp Val Val Ile Tyr Phe Ser Gly
           35                  40                  45
   Asp Asp Ser Cys Ser Leu Gln Val Pro Gly Ser Thr Lys Gly Leu Ile
       50                  55                  60
   Cys His Cys Arg Val Leu Tyr Cys Ile Phe Gly Glu His Leu Gly Gly
   65                  70                  75                  80
   Thr Cys Phe Ile Leu Gly Glu Arg Tyr Pro Ile Cys Cys Tyr
                   85                  90
**(2) INFORMATIONS POUR LA SEQ ID NO: 4:**
   (i) CARACTERISTIQUES DE LA SEQUENCE:
      (A) LONGUEUR: 19 ACIDES AMINES
      (B) TYPE: ACIDE AMINE
      (C) NOMBRE DE BRINS: SIMPLE
      (D) CONFIGURATION: LINEAIRE
   (ii) TYPE DE MOLECULE: PEPTIDE SIGNAL
   (vi) ORIGINE:
      (A) ORGANISME: Homo sapiens
   (xi) SEQUENCE DESCRIPTION: SEQ ID NO: 4:
   Met Arg Thr Leu Thr Leu Leu Ser Ala Phe Leu Leu Val Ala Leu Gln
   1               5                   10                  15
   Ala Trp Ala
**(2) INFORMATIONS POUR LA SEQ ID NO: 5:**
   (i) CARACTERISTIQUES DE LA SEQUENCE:
      (A) LONGUEUR: 44 ACIDES AMINES
      (B) TYPE: ACIDE AMINE
      (C) NOMBRE DE BRINS: SIMPLE
      (D) CONFIGURATION: LINEAIRE
   (ii) TYPE DE MOLECULE: REGION PRO
   (vi) ORIGINE:
      (A) ORGANISME: Homo sapiens
   (xi) SEQUENCE DESCRIPTION: SEQ ID NO: 5:
   Glu Pro Leu Gln Ala Arg Ala His Glu Met Pro Ala Gln Lys Gln Pro
   1               5                   10                  15
   Pro Ala Asp Asp Gln Asp Val Val Ile Tyr Phe Ser Gly Asp Asp Ser
               20                  25                  30
   Cys Ser Leu Gln Val Pro Gly Ser Thr Lys Gly Leu
           35                  40
**(2) INFORMATIONS POUR LA SEQ ID NO: 6:**
   (i) CARACTERISTIQUES DE LA SEQUENCE:
      (A) LONGUEUR: 31 ACIDES AMINES
      (B) TYPE: ACIDE AMINE
      (C) NOMBRE DE BRINS: SIMPLE
      (D) CONFIGURATION: LINEAIRE
   (ii) TYPE DE MOLECULE: PEPTIDE MATURE
   (vi) ORIGINE:
      (A) ORGANISME; Homo sapiens
   (xi) SEQUENCE DESCRIPTION: SEQ ID NO: 6:
   Ile Cys His Cys Arg Val Leu Tyr Cys Ile Phe Gly Glu His Leu Gly
   1               5                   10                  15
   Gly Thr Cys Phe Ile Leu Gly Glu Arg Tyr Pro Ile Cys Cys Tyr
               20                  25                  30